(19) **Europäisches Patentamt / European Patent Office / Office européen des brevets**

(11) **EP 4 736 997 A1**

(12) **EUROPEAN PATENT APPLICATION**
published in accordance with Art. 153(4) EPC

(43) Date of publication:
06.05.2026 Bulletin 2026/19

(21) Application number: 24832079.8

(22) Date of filing: 28.06.2024

(51) International Patent Classification (IPC):
$B01F\ 23/45^{(2022.01)}$  $A61K\ 9/127^{(2025.01)}$
$B01F\ 25/43^{(2022.01)}$  $B01F\ 33/301^{(2022.01)}$
$B01F\ 35/22^{(2022.01)}$  $B01F\ 35/71^{(2022.01)}$
$B01F\ 35/75^{(2022.01)}$  $B01F\ 35/92^{(2022.01)}$
$B01F\ 35/213^{(2022.01)}$  $B01F\ 35/221^{(2022.01)}$
$B01J\ 2/10^{(2006.01)}$  $C12N\ 15/88^{(2006.01)}$
$B01F\ 101/22^{(2022.01)}$

(52) Cooperative Patent Classification (CPC):
A61K 9/127; B01F 23/45; B01F 25/43;
B01F 33/301; B01F 35/213; B01F 35/22;
B01F 35/221; B01F 35/71; B01F 35/75;
B01F 35/92; B01J 2/10; C12N 15/88; B01F 2101/22

(86) International application number:
PCT/JP2024/023467

(87) International publication number:
WO 2025/005221 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 29.06.2023 JP 2023107138

(71) Applicants:
• The University of Osaka
Osaka 565-0871 (JP)

• Shionogi Pharma Co., Ltd.
Settsu-shi, Osaka 566-0022 (JP)

(72) Inventors:
• MATSUZAKI, Takashi
Suita-shi, Osaka 565-0871 (JP)
• FUJIKI, Daichi
Settsu-shi, Osaka 566-0022 (JP)

(74) Representative: J A Kemp LLP
80 Turnmill Street
London EC1M 5QU (GB)

(54) **APPARATUS AND METHOD FOR MANUFACTURING COMPOSITE**

(57) The present disclosure provides an apparatus and a method for manufacturing a composite. In one aspect, the present disclosure provides a method for manufacturing a formulation comprising a first component and a second component. This method includes: a step for preparing a series of flow paths in which a first flow path through which a first fluid flows at a first flow velocity and a second flow path through which a second fluid flows at a second flow velocity are combined at a merging portion to form a third flow path through which a third fluid flows at a third flow velocity; a step in which the first component is injected into the first flow path at a first time; and a step in which the second component is injected into the second flow path at a second time. The first time and the second time are set so that the first component and the second component reach the merging portion at a time when the components are mixed.

EP 4 736 997 A1

[Fig. 1A]

FIG. 1A

**Description**

[Technical Field]

The present disclosure relates to an apparatus and a method for producing a composite. More particularly, the present disclosure relates to a technique for producing a composite by mixing fluids flowing from at least two flow channels.

[Background Art]

An "in-line liposome production technology" in which liposomes or lipid nanoparticles (LNPs) are continuously formed in a closed tube has been attracting attention as a technology for producing sterile liposomes, and a technology for adjusting a concentration of the thus-formed liposomes in an in-line manner has been developed (see Patent Literature 1). However, various parameters such as a flow velocity need to be adjusted in order to obtain suitable lipid particles for a factor such as a particle size, and parameters set for preparation of a small amount of a sample at a laboratory level often differ from those suitable for mass production for product supply.
Therefore, in the past, conditions of manufacture for product supply had to be separately adjusted with many materials consumed.

[Citation List]

[Patent Literature]

[PTL 1]
PCT International Publication No. WO 2016/024510

[Summary of Invention]

[Solution to Problem]

As a result of extensive studies to find a method for reflecting conditions for preparation of a small amount of a lipid particle sample into conditions of manufacture for product supply in order to reduce a need for additional studies to determine the conditions of manufacture for product supply, the present inventors have completed an apparatus and a method of the present disclosure. Therefore, the present invention provides an apparatus and a method for producing drug-loaded lipid particles by mixing fluids flowing from at least two flow channels.
Therefore, the present disclosure provides the following items.

(Item 1)

A method for producing a formulation including:

a first component; and
a second component, the method including:

preparing a series of flow channels including a first flow channel through which a first fluid flows at a first flow velocity, a second flow channel through which a second fluid flows at a second flow velocity, and a third flow channel through which a third fluid flows at a third flow velocity, the third flow channel being formed by combining the first flow channel and the second flow channel at a combination portion;
injecting a sample including the first component into the first flow channel at a first time; and
injecting a sample including the second component into the second flow channel at a second time,
the first time and the second time being set so that the first component and the second component reach the combination portion at a time when the first component and the second component are mixed.

(Item 2)

The method according to any of the above items, in which the first component is injected into a middle of the first flow channel and the second component is injected into a middle of the second flow channel.

(Item 3)

The method according to any of the above items, in which a third time is set based on the first time and the second time.

(Item 4)

The method according to any of the above items, in which the first flow velocity or the second flow velocity exceeds about 2.4 mL/min.

(Item 5)

The method according to any of the above items, in which the series of flow channels includes a fourth flow channel through which a sample including a third component flows; the method further includes

injecting the third component into the fourth flow channel at the third time; and
the first time, the second time, and the third time are set so that the first component, the second component, and the third component reach the combination portion at a time when the first component, the second component, and the third component are mixed.

(Item 6)

The method according to any of the above items, in which the third flow channel and the fourth flow channel are combined at an additional combination portion downstream of the combination portion.

(Item 7)

The method according to any of the above items, in which the first flow channel, the second flow channel, and the fourth flow channel are combined at the combination portion.

(Item 8)

The method according to any of the above items, further including detecting a combination state of the first component and the second component downstream of the combination portion.

(Item 9)

The method according to any of the above items, in which the combination state to be detected is a state selected from the group consisting of a concentration of at least one of the first component and the second component, a particle diameter of particles in a flow, and a state of a chemical bond between the first component and the second component.

(Item 10)

The method according to any of the above items, in which the first flow channel is in a liquid-feeding state in the injection of the sample including the first component into the first flow channel and the second flow channel is in a liquid-feeding state in the injection of the sample including the second component into the second flow channel.

(Item 11)

The method according to any of the above items, in which, in the injection of the sample including the first component into the first flow channel and in the injection of the sample including the second component into the second flow channel, retention times taken for the sample including the first component and the sample including the second component to be introduced into sample injection portions and then injected into the flow channels are independently within 1 minute.

(Item 12)

The method according to any of the above items, in which times taken for the first component and the second component to be injected and reach the combination portion are independently within 2 seconds.

(Item 13)

The method according to any of the above items, in which 1000 μg or less of the first component is injected in the injection of the first component into the first flow channel and 1000 μg or less of the second component is injected in the injection of the second component into the second flow channel.

(Item 14)

The method according to any of the above items, in which a fourth fluid flowing through a fifth flow channel is combined with the third fluid at the additional combination portion to form a sixth flow channel through which a fifth fluid flows.

(Item 15)

The method according to any of the above items, in which the combination portion includes a T-mixer, a microflow channel device, or a static mixer.

(Item 16)

The method according to any of the above items, in which the third flow channel includes a mixer.

(Item 17)

The method according to any of the above items, in which the mixer on the third flow channel is a static mixer.

(Item 18)

The method according to any of the above items, in which each of the first flow channel and the second flow channel includes a plurality of branched flow channels, and the combination portion includes a plurality of branched flow combination portions at which the branched flow channels from the first flow channel and the branched flow channels from the second flow channel are combined.

(Item 19)

The method according to any of the above items, in which the first flow channel and/or the second flow channel is branched to form the branched flow channel.

(Item 20)

The method according to any of the above items, in which the branched flow channels downstream of the branched flow combination portion combine with each other in the third flow channel.

(Item 21)

The method according to any of the above items, further including removing a fraction including a composite of the first component and the second component from a collection portion of the third flow channel at a third time.

(Item 22)

The method according to any of the above items, in which the collection portion is positioned in a middle of the third flow channel.

(Item 23)

The method according to any of the above items, further including dispensing the fraction to a receiving portion.

(Item 24)

The method according to any of the above items, in which the dispensing includes dispensing a plurality of the fractions to the receiving portion including a plurality of compartments.

(Item 25)

The method according to any of the above items, the method including testing the plurality of the fractions.

(Item 26)

The method according to any of the above items, in which a time when the fraction is removed from the collection portion is controlled based on a time and a flow velocity at which the component is injected into the flow channel.

(Item 27)

An apparatus for injecting a plurality of samples into a plurality of flow channels at a time when a first component and a second component are mixed, the apparatus including:

a first flow channel through which a first fluid flows;
a first injection portion from which a sample including the first component is injected into the first flow channel;
a second flow channel through which a second fluid flows;
a second injection portion from which a sample including the second component is injected into the second flow channel;
a combination portion in which the first flow channel and the second flow channel combine with each other;
a third flow channel through which a third fluid flows and which extends from the combination portion; and
a control portion,
the control portion being configured to control a time when the first injection portion injects the first component into the first flow channel and a time when the second injection portion injects the second component into the second flow channel.

(Item 28)

The apparatus according to any of the above items, in which the first injection portion is positioned in a middle of the first flow channel and the second injection portion is positioned in a middle of the second flow channel.

(Item 29)

The apparatus according to any of the above items, in which the first injection portion is a valve capable of injecting the first component into the first flow channel within 5 seconds, and the second injection portion is a valve capable of injecting the second component into the second flow channel within 5 seconds.

(Item 30)

The apparatus according to any of the above items, in which at least one of the first injection portion and the second injection portion can switch flow channels within about 500 ms.

(Item 31)

The apparatus according to item 19, further including:

a fourth flow channel through which the third fluid flows; and
a third injection portion from which a sample including a third component is injected into the fourth flow channel,
in which a series of flow channels includes the fourth flow channel, and
in which the control portion is configured to control a time when the first injection portion injects the first component into the first flow channel, a time when the second injection portion injects the second component into the second flow channel, and a time when the third injection portion injects the third component into the fourth flow channel.

(Item 32)

The apparatus according to any of the above items, in which the apparatus includes a detector downstream of the combination portion.

(Item 33)

The apparatus according to any of the above items, in which the first injection portion includes a valve with a sample loop having a volume of 5 mL or less and the second injection portion includes a valve with a sample loop having a volume of 5 mL or less.

(Item 34)

The apparatus according to any of the above items, in which a pump through which the first fluid and the second fluid flow is a plunger pump.

(Item 35)

The apparatus according to any of the above items, in which a back pressure on the pump through which the first fluid and the second fluid flow is 1 MPa or more.

(Item 36)

The apparatus according to any of the above items, further including:

a fourth flow channel through which a fourth fluid flows;
an additional combination portion in which the fourth flow channel and the third flow channel combine with each other; and
a fifth flow channel through which a fifth fluid flows and which extends from the additional combination portion.

(Item 37)

The apparatus according to any of the above items, in which the third flow channel includes a collection portion and, further, the collection portion includes a collection device.

(Item 38)

The apparatus according to any of the above items, further including a thermostatic bath that maintains at least a portion of the first, second, and third flow channels, the first and second injection portions, and the combination portion at a constant temperature.

(Item 39)

The apparatus according to any of the above items, in which the control portion further controls a collection time of the collection device.

(Item 40)

The apparatus according to any of the above items, in which the control portion controls at least one of a collection start time and a time interval between collections of the collection device.

(Item 41)

The apparatus according to any of the above items, in which the collection portion is positioned in a middle of the third flow channel.

(Item 42)

The apparatus according to any of the above items, in which the collection device includes a valve.

(Item 43)

The apparatus according to any of the above items, in which the valve is a slide solenoid valve.

(Item 44)

The apparatus according to any of the above items, further including a receiving portion that receives a fraction removed from the collection device.

(Item 45)

The apparatus according to any of the above items, in which the receiving portion includes a plurality of compartments that receive a plurality of fractions.

(Item 46)

A program for injecting a plurality of components into a plurality of flow channels connected to a combination portion, the program being executed in an apparatus including a processor, the program causing the processor to implement a processing including:

receiving information about a first flow channel and information about a second flow channel;
determining a first time at which a sample including a first component is injected into the first flow channel and a second time at which a sample including a second component is injected into the second flow channel based on the information about the first flow channel and the information about the second flow channel, the first time and the second time being a time is set so that the first component injected into the first flow channel and the second component injected into the second flow channel reach a combination portion at a time when the first component and the second component are mixed;
instructing an injection device to inject the sample including the first component into the first flow channel at the first time; and
instructing an injection device to inject the sample including the second component into the second flow channel at the second time.

(Item 47)

The program according to any of the above items, in which the processing further includes

receiving information about a third flow channel downstream of the combination portion;
determining a third time at which a fraction is removed from the third flow channel based on the information about the third flow channel; and
instructing a collection device to remove the fraction from the third flow channel at the third time.

(Item 48)

A recording medium storing a program for causing an apparatus including a processor to execute a method for injecting a plurality of components into a plurality of flow channels connected to a combination portion, the method including:

receiving information about a first flow channel and information about a second flow channel;
determining a first time at which a sample including a first component is injected into the first flow channel and a second time at which a sample including a second component is injected into the second flow channel based on the information about the first flow channel and the information about the second flow channel, the first time and the second time being times set so that the first component injected into the first flow channel and the second component injected into the second flow channel reach a combination portion at a time when the first component and the second component are mixed;

instructing an injection device to inject the sample including the first component into the first flow channel at the first time; and
instructing an injection device to inject the sample including the second component into the second flow channel at the second time.

(Item 49)

The recording medium according to any of the above items, in which the method further includes

receiving information about a third flow channel downstream of the combination portion;
determining a third time at which a fraction is removed from the third flow channel based on the information about the third flow channel; and
instructing a collection device to remove the fraction from the third flow channel at the third time.

(Item 50)

A method for screening a condition for producing drug-loaded lipid particles, the method including:

preparing a series of flow channels including a first flow channel through which a first fluid flows at a first flow velocity, a second flow channel through which a second fluid flows at a second flow velocity, and a third flow channel through which a third fluid flows at a third flow velocity, the third flow channel being formed by combining the first flow channel and the second flow channel at a combination portion;

(1) injecting a sample including a candidate lipid into the first flow channel at a first time;
(2) injecting a sample including a candidate drug into the second flow channel at a second time;
(3) removing a fraction including particles of the candidate lipid from a collection portion of the third flow channel at a third time; and

evaluating the particles of the candidate lipid,
the first time and second time being set so that the candidate lipid and the candidate drug simultaneously reach the combination portion.

(Item 51)

The method according to any of the above items, further including preparing a plurality of the particles of the candidate lipid, comparing the plurality of the particles of the candidate lipid, and selecting hit lipid particles by repeating (1) to (3).

(Item 52)

A method for producing the hit lipid particles according to any of the above items under the condition set in the method for screening according to any of the above items.

(Item 53)

The method for producing the hit lipid particles according to any of the above items, in which a frequency with which (1) to (3) are performed is increased, thereby increasing an amount of the hit lipid particles to be produced.
In the present disclosure, it is intended that the above-mentioned one or more features may be provided in further combinations in addition to the explicit combinations. Those skilled in the art will recognize further embodiments and advantages of the present disclosure upon reading and understanding the following detailed description, if necessary.

[Advantageous Effects of Invention]

According to the present disclosure, appropriate reaction conditions (e.g., conditions for forming lipid particles) can be determined with consumption of a small amount of a material, and a quantity of production can be easily scaled up using the conditions, thus reducing material consumption and providing flexibility to meet the need of providing different products.

[Brief Description of Drawings]

[FIG. 1A] An exemplary apparatus (system) of the present disclosure is shown. 101: Autosampler, 102: Injection portion, 103: Pump, 104: Flow channel, 105: Combination portion, 106: Control portion, 107: Collection device, 108: Valve, 109: Receiving portion.

[FIG. 1B] A variant of the apparatus (system) in FIG. 1A is shown. 104c': Branched flow channel, 105': Branched flow combination portion.

[FIG. 2] An exemplary apparatus (system) of the present disclosure is shown. This shows an embodiment including a computer (110) and a detector (111) in addition to the configuration shown in FIG. 1.

[FIG. 3A] A block diagram is shown in which configuration of a control unit for a particle size of lipid particles in one embodiment of the present invention is shown by function.

[FIG. 3B] A block diagram is shown in which configuration of a control unit for a particle size of lipid particles in one embodiment of the present invention is shown by function.

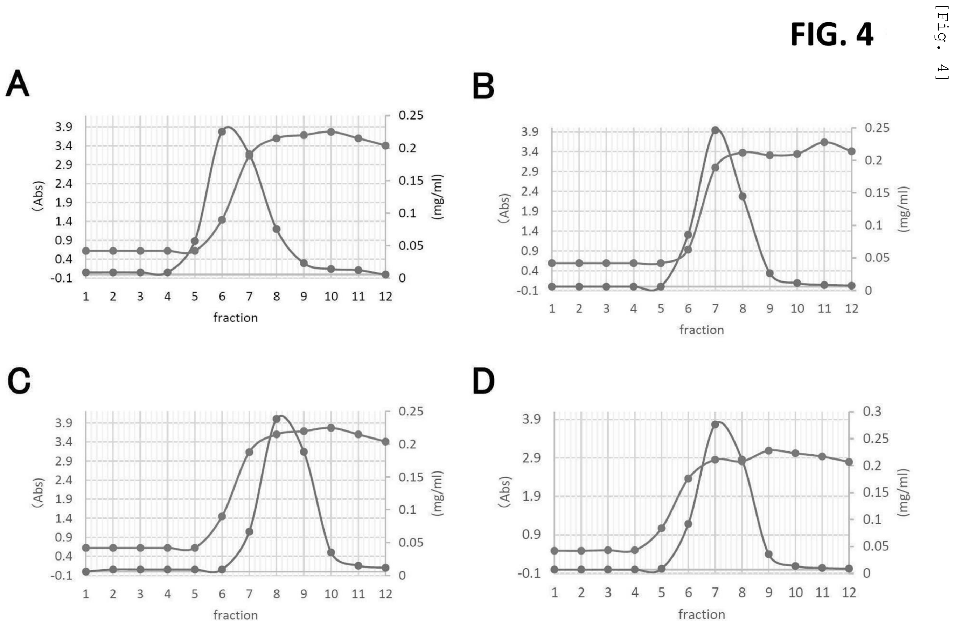

[FIG. 4] An examination to achieve proper mixing of two samples at simultaneous timing of injection is shown. Each graph shows absorbance of a nucleic acid and a lipid in consecutive fractions acquired. FIG. 4A shows results when two flow channels were fitted with pipes having the same pipe inner diameter and pipe length. FIG. 4B shows results when a pipe with an inner diameter of 0.381 mm and a length of 76 cm (87 μL) was added to the flow channel into which a nucleic acid sample was injected. FIG. 4C shows results when the pipe added to the flow channel into which the nucleic acid sample was injected was switched to a pipe with an inner diameter of 0.381 mm and a length of 134 cm (153 μL). FIG. 4D shows results when a pipe from a valve to a mixer in a flow channel into which a lipid sample was injected was changed to a smaller-sized pipe having an inner diameter of 0.18 mm and a length of 6 cm under the condition that the pipe with an inner diameter of 0.381 mm and a length of 76 cm (87 μL) was added to the flow channel into which the nucleic acid sample was injected.

[FIG. 5] An examination to achieve proper mixing of two samples when pipes having the same pipe inner diameter and pipe length are used in flow channels is shown. Each graph shows absorbance of a nucleic acid and a lipid in consecutive fractions acquired. FIG. 5A shows results if a signal to inject the samples is sent to two valves simultaneously. FIG. 5B shows results if a time when a nucleic acid sample is injected delayed by 100 msec from a time when a lipid sample is injected.

[FIG. 6] Changes in nucleic acid and lipid concentrations in fractions collected when a valve is changed are shown. A vertical axis shows absorbance at a wavelength of 260 nm or 600 nm, and a horizontal axis shows the number of a series of fractions acquired.

[FIG. 7] Changes in a particle size distribution of lipid particles in fractions collected when a valve is changed are shown.

[FIG. 8] An overview of an apparatus configuration in Example 6 using four pumps to mix three components is shown.

[Description of Embodiments]

The present disclosure will be described with reference to the best mode thereof. It should be understood that throughout this specification, singular expressions also include the concept of their plural forms, unless otherwise noted. Thus, it should be understood that singular articles (e.g., "a", "an", "the", etc. in English) also include the concept of their plural forms, unless otherwise noted. It should also be understood that terms used herein are to be used in the sense normally used in the art, unless otherwise noted. Therefore, unless otherwise defined, all technical and scientific terms used herein have the same meaning as commonly understood by those skilled in the art to which the present disclosure pertains. In the event of a conflict, this specification (including definitions) shall prevail. Definitions and/or basic technical details of terms specifically used herein will be described as appropriate.

(Definitions)

As used herein, the phrase "flow channel" refers to a structure that is commonly tubular and through which a fluid flows. The flow channel herein typically refers to a series of structures from a mechanism that draws a fluid into a tube such as a pump to a combination portion, from one combination portion to another combination portion, or from a mechanism that draws a fluid into a tube or a combination portion to an outlet from which the fluid is discharged. For example, even if a mechanism capable of temporarily interrupting a flow such as a valve or a collection portion exists along the way, it can be considered a single flow channel. The flow channel does not necessarily have to be composed of a single member, but may include a member connecting, for example, pipes. Typically, a single flow channel is in fluid communication as a whole. Typically, the flow channel has no regions where a fluid is in contact with an outside other than an inflow port or an outflow port.

As used herein, the phrase "combination portion" refers to a region into which a fluid flows from a plurality of flow channels, typically connected to one or more flow channels through which a fluid flowed from a combination portion flows. The combination portion may be described as a mixer because a plurality of fluids is usually mixed at the combination portion.

As used herein, the phrase "flow velocity" refers to a velocity at which a solution flows through a tube and is a quantity that may be expressed in any of dimensions (distance)/(time) or (volume)/(time) herein.

As used herein, the phrase "retention time" refers to a time taken for a solution to transfer from one region to another region. For example, if a region A is connected to a region B via a liquid-feeding tube C, a retention time from the region A to the region B can be calculated as (Length of liquid-feeding tube)/(Flow velocity [cm/min]).

As used herein, the term "composite" is an entity formed by contact of a plurality of components, such as drug-loaded lipid particles each composed of a lipid and a drug, a product formed by a chemical reaction between different compounds, or a composite formed between different proteins.

As used herein, the term "composite" or "composite molecule" means any construct containing two or more parts. For example, if one part is a polypeptide, the other part may be a polypeptide or another substance (e.g., a sugar, a lipid, a nucleic acid, other hydrocarbons, etc.). The two or more parts constituting a composite herein may be bound by a covalent bond or by another bond (e.g., a hydrogen bond, an ionic bond, a hydrophobic interaction, a van der Waals force, etc.). If two or more parts are polypeptides, the composite may also be referred to as a chimeric polypeptide. Thus, the "composite" herein includes a molecule formed by linking a plurality of types of molecules such as a polypeptide, a polynucleotide, a lipid, a sugar, and a small molecule.

As used herein, the phrase "lipid particles" refers to lipid vesicles each formed from a lipid bilayer containing a lipid molecule, specifically, vesicles each having a space separated from an outside by a lipid bilayer created based on polarity of a hydrophobic group and a hydrophilic group of a lipid molecule. The lipid particles may be small unilamellar vesicles (SUVs) with a single lipid bilayer membrane, multilamellar vesicles (MLVs) with a plurality of layers, or lipid nanoparticles with a lipid-filled core covered with a lipid monolayer. The bilayer membrane is composed of two monolayer lipid membranes with a hydrophobic "tail" region and a hydrophilic "head" region. A membrane bilayer has a structure so that the hydrophobic (nonpolar) "tail" of a lipid monolayer faces a center of the bilayer, while the hydrophilic "head" faces an aqueous phase. The lipid bilayer membrane is similar to a cellular membrane that makes up a living organism and is therefore easily accepted into an in vivo environment. Therefore, a liposome is sometimes used in a drug delivery system (DDS). For example, a drug can be encapsulated in a liposome to transport the drug to a predetermined site in vivo. Although a liposome of the present disclosure is particularly suitable for a pharmaceutical application, its use is not limited and it may be used in applications such as food, cosmetics, agriculture, imaging, etc.

When a lipid particle is "loaded" with a drug herein, the drug is held inside and/or on a surface of the lipid particle. When the lipid particle is "loaded" with the drug, at least a portion of the drug is held in a lipid bilayer membrane inside or on a surface of the lipid particle. The drug may be present in an aqueous phase within the lipid particle, immobilized by, for example, electrostatic interaction on a surface layer of a lipid layer, or partially or entirely contained within a lipid layer.

As used herein, the term "lipid" is used in a usual sense in the art and refers to a substance with a hydrophobic portion such as a long-chain fatty acid or a hydrocarbon chain. Examples of the lipid include phosphatidylcholine (soy lecithin, hydrogenated soy lecithin, egg yolk lecithin, etc.), phosphatidylserine, phosphatidylethanolamine, phosphatidylinositol, phosphasphingomyelin, phosphatidic acid, a long-chain alkyl phosphate, ganglioside, a glycolipid, phosphatidylglycerol, cholesterol (cholesterol, phytosterol (sitosterol, stigmasterol, fucosterol, spinasterol, brassicasterol), lanosterol, ergosterol, a fatty acid ester thereof, etc.), tocopherol, steroid, a fatty acid, and a fatty acid ester of glycerol, etc. The lipid can also be classified into an amphiphilic lipid (a phospholipid, an aminolipid, a sphingolipid, a sphingoglycolipid, diacylglycerol, and β-acyloxy acid, etc.), an anionic lipid (phosphatidylglycerol, cardiolipin, diacylphosphatidylserine, diacylphosphatidic acid, N-dodecanoylphosphatidylethanolamine, N-succinylphosphatidylethanolamine, N-glutarylphosphatidylethanolamine, lysylphosphatidylglycerol, and another anionic modified group attached to a neutral lipid, etc.), a cationic lipid (N,N-dioleoyl-N,N-dimethylammonium chloride ("DODAC"), N-(2,3-oleoyloxy)propyl-N,N,N-trimethylammonium chloride ("DOTMA"), N,N-distearyl-N,N-dimethylammonium bromide ("DDAB"), N-(2,3-oleoyloxy)propyl-N,N,N-trimethylammonium chloride ("DOTAP"), 3-(N-(N',N'-dimethylaminoethane)-carbamoyl)cholesterol ("DC-Chol"), and N(1,2-dimyristiroxyprop-3-yl)N,N-dimethylhydroxyethylammonium bromide ("DMRIE"), etc.). The lipid also includes an ionized lipid such as D-Lin-MC3-DMA, ALC-0315, and SM-102.

As used herein, the terms "drug", "agent", and "factor" (all equivalent to "agent" in English) are used interchangeably in a broad sense and may be any substance or another element (e.g., an energy such as light, radioactivity, heat, electricity) as long as the intended purpose can be achieved. Examples of such a substance include, but are not limited to, a protein (including an antibody, etc.), a polypeptide, an oligopeptide, a peptide, a polynucleotide, an oligonucleotide, a nucleotide, a nucleic acid (including, for example, DNA such as cDNA and genomic DNA, RNA such as mRNA), a polysaccharide, an oligosaccharide, a lipid, an organic small molecule (e.g., a hormone, a ligand, a transmitter, an organic small molecule, a molecule synthesized in combinatorial chemistry, a small molecule that can be used as a medicament, etc), and a complex molecule thereof.

Generally, a composition, a medicament, an agent (a therapeutic agent, a prophylactic agent, etc.) of the present disclosure includes a therapeutically effective amount of a pharmaceutical or active ingredient, and a pharmaceutically acceptable carrier or excipient. As used herein, the phrase "pharmaceutically acceptable" means that it is approved by a governmental regulatory agency or listed in a pharmacopoeia or other generally accepted pharmacopoeia for use in animals and, more specifically, in humans.

As used herein, the term "drug" is used in a usual sense in the art and refers to anything that exerts some physiological effect when administered to an organism. Examples thereof include a protein (including an enzyme, an antibody, etc.), a peptide, a nucleic acid (DNA, mRNA, siRNA, miRNA), a vector, a viral particle, a plasmid, a toxin, a saccharide (an oligosaccharide and a polysaccharide), a polymeric compound, an anticancer agent, an antibiotic, an enzyme, an antioxidizing agent, a lipid uptake inhibitor, a hormone, an anti-inflammatory agent, a steroid, a vasodilator, a angiotensin converting enzyme inhibitor, a angiotensin receptor antagonist, a smooth muscle cell proliferation and migration inhibitor, an platelet aggregation inhibitor, an anticoagulant, a chemical mediator release inhibitor, a vascular endothelial cell proliferation stimulator or inhibitor, an aldose reductase inhibitor, a mesangial cell proliferation inhibitor, a lipoxygenase inhibitor, a immunosuppressive agent, an immunostimulant, an anti-rheumatic drug, an anti-inflammatory enzyme preparation, an arthrifuge drug, an antihistamine, a chemical transmitter release inhibitor, an antiviral agent, a Maillard reaction inhibitor, an amyloidosis inhibitor, a nitric oxide synthesis inhibitor, an advanced glycation endproduct (AGF) inhibitor, a hemoglobin, a radical scavenger, a glycosaminoglycan and a derivative thereof; a corticosteroid such as prednisolone, methylprednisolone, dexamethasone and a derivative thereof; a non-steroidal anti-inflammatory agent such as aspirin, indomethacin, ibuprofen, mefenamic acid, phenylbutazone; a mesangial cell proliferation inhibitor such as heparin and a low molecular weight heparin; an immunosuppressant such as cyclosporine; an angiotensin converting enzyme (ACE) inhibitor such as captopril; an advanced glycation endoproduct (AGE) inhibitor such as methylguanidine; a TGF-$\beta$ antagonist such as biglycan and decorin; a protein kinase C (PKC) inhibitor; a prostaglandin preparation such as PGE1 and PGI2; a peripheral vasodilator such as papaverine, nicotinic acid, tocopherol, and a Ca antagonist; a phosphodiesterase inhibitor; an antithrombotic drug such as ticlopidine and aspirin; an anticoagulant such as warfarin, heparin, and an antithrombin agent; a thrombolytic drug such as a urokinase; a chemical mediator release inhibitor, an antibiotic, an antioxidizing agent, an enzyme, a lipid uptake inhibitor, a hormone; a radical scavenger such as vitamin C, vitamin E, and SOD; an antisense oligonucleotide that inhibits mesangial cell proliferation, etc.

As used herein, the phrase "particle size" or "particle diameter" is a measure to be used to describe a size of a particle, as used in a usual sense in the art, and is a convenient value corresponding to a diameter of a particle, assuming it is a perfect sphere. A particle size of a liposome may be measured by any method known in the art, for example, a freeze-fracture method using a transmission electron microscope (TEM) and a dynamic light scattering method utilizing, for example, Malvern Zetasizer. As used herein, the term "average particle diameter" may be used to refer to either a number average particle diameter, a volume average particle diameter, or a Z-average particle diameter, but unless otherwise specified, it refers to a Z-average particle diameter calculated from a particle diameter measurement. As used herein, the term "particle size distribution" is used in a usual sense in the art and refers to a spread of particle sizes. A polydispersity index (PDI) is used as a measure of the particle size distribution.

As used herein, the term "measurement" is used in a usual sense in the art and means measurement to determine an amount of a certain target. As used herein, the term "detection" is used in a usual sense in the art and refers to testing to find a substance or a component, etc., the term "identification" refers to an action of searching for an attribution of a certain target among the existing classifications regarding the target. When used in the art of chemistry, the term refers to determination of identity of a substance of interest as a chemical substance (e.g., determination of its chemical structure), and the term "quantification" refers to determination of an abundance of a substance of interest.

As used herein, the term "means" refers to anything that can be an arbitrary tool to achieve a purpose (e.g., detection).

As used herein, the term "program" is used in a usual sense in the art and is an ordered description of a processing to be executed by a computer, and is treated as a "product" under the law. All computers operate according to a program. In modern computers, a program is expressed as data and is stored on a recording medium or a storage device.

As used herein, the phrase "recording medium" is a recording medium that stores a program for executing the present disclosure, and the recording medium may be any medium as long as it can record the program. For example, the recording medium may be a ROM, an HDD, or a magnetic disk that can be internally housed, or an external storage device such as a flash memory, for example, a USB memory, etc., but is not limited thereto.

As used herein, the term "system" refers to a configuration that executes a method or a program of the present disclosure, and essentially means a scheme or organization for carrying out a purpose and composed of a plurality of elements systematically organized and interacting with each other. The system refers to the overall configuration of hardware, software, an operating system, a network, etc., in the art of a computer.

The term "about" as used herein with regard to a quantitative measurement other than a temperature refers to an indicated value plus or minus 10%, or in some cases, an indicated value plus or minus 20%. The term, as used herein with regard to a temperature, refers to an indicated value plus or minus 5°C.

(Preferred embodiment) Preferred embodiments of the present disclosure will be described. It is understood that embodiments provided below are given for a better understanding of the present disclosure and the scope of the present disclosure should not be limited to the following description. Therefore, it is clear that those skilled in the art may make modifications as appropriate within the scope of the present disclosure, taking into account the description herein. It is understood that the following embodiments of the present disclosure may also be used alone or in combination.

Although the present disclosure includes descriptions of various aspects of a method, an apparatus, a system, etc., a description of one aspect applies equally to other aspects. For example, a description of a method (e.g., flow velocity) could simultaneously be considered a description of an operating condition of an apparatus or a description of an instruction to be coded into a program.

(Method)

In one aspect, the present disclosure provides a method for producing a formulation including a first component and a second component. The method includes preparing a series of flow channels including a first flow channel through which a first fluid flows at a first flow velocity, a second flow channel through which a second fluid flows at a second flow velocity, and a third flow channel through which a third fluid flows at a third flow velocity, the third flow channel being formed by combining the first flow channel and the second flow channel at a combination portion; injecting a sample including the first component into the first flow channel at a first time; and injecting a sample including the second component into the second flow channel at a second time. The first time and the second time are set so that the first component and the second component reach the combination portion at a time when the first component and the second component are mixed. In one embodiment, the method further includes injecting a sample including a third component into a fourth flow channel at a third time, the first time, the second time, and the third time may be set so that the first component, the second component, and the third component reach the combination portion at a time when the first component, the second component, and the third component are mixed, and more components may be mixed as well. In one embodiment, the fourth flow channel through which the third component flows may combine with the third flow channel at an additional combination portion and the third time at which the sample including the third component is injected into the fourth flow channel is set to reach the combination portion at a time when a composite of the first component and the second component are mixed with the third component. Such an operation and feature of the fourth flow channel are understood in the same manner as for the first flow channel and the second flow channel.

By injecting the sample while the fluid keeps on flowing through the flow channel, the components can be mixed with each other to form a composite under conditions that mimic mass production with only a small amount of the components being consumed. Under conditions for mass production, a time when the sample is injected needs to be controlled so that the components are mixed with each other because a flow velocity is usually high. As used herein, the phrase "timing of mixing" or a similar description of a time when different components flowing through different flow channels are mixed with each other indicates a presence and/or a range of time during which at least portions of components injected at a time are brought into contact with each other. The time when the sample is injected (typically, a time point at which an injection portion such as a valve begins to operate) such as the first time and the second time can be determined depending on the timing of mixing. The time when the sample is injected such as the first time can vary depending on a factor such as a set value of a flow velocity or a flow channel (an inner diameter and a length) and the first time and the second time may result in the same as each other depending on the set value of the factor, in some embodiments. A timing of injection will be detailed below.

A sample can have a constant volume when the sample is injected into a flow channel and, therefore, a component in the sample moves so that the component is present in a portion with a certain length in the flow channel. By injecting the sample into the flow channel, the sample comes into contact with a fluid flowing through the flow channel in front of and behind the sample, even if the component is uniformly dispersed in the sample before injection. Therefore, the component diffuses at a contact surface between the sample and the fluid (if the sample and the fluid are miscible, a clear contact surface is not formed), a concentration of the component in the flow channel becomes heterogeneous, and thus, usually a mountain-like concentration distribution may be formed in a length direction of the flow channel. Typically, the first time and the second time can be selected so that a duration in which fractions of a fluid having a concentration of 50% or more of a component concentration in a sample including the first component passes through an inflow port of the combination portion at least partially overlaps with that of a sample including the second component (for example, at least about 50, about 60, about 70, about 80, about 90, or about 100% of a shorter duration overlaps a longer duration). The first time and the second time can be determined based on flow velocities of the first flow channel and the second flow channel and a length from each of the injection portion for the sample including the first component and the injection portion for the sample including the second component to the combination portion. In one embodiment, the first time and the second time may be determined based on flow velocities ($V_1$ and $V_2$) of the first flow channel and the second flow channel, injection volumes of the sample including the first component and the sample including the second component ($W_1S$ and $W_2S$), and

internal volumes ($W_1L$ and $W_2L$) of the flow channels from the injection portions to the combination portion. In one embodiment, inflow times from inflow start times of the first component and the second component into the combination portion ($T_10$ and $T_20$) to inflow termination times ($T_1E$ and $T_2E$) (inflow time of the first component $T_1$: $T_1E$ to $T_10$ and inflow time of the first component $T_2$: $T_2E$ to $T_20$) are set so that a shorter inflow time is encompassed within a longer inflow time. For example, the following condition is exemplified.

[Mathematical 1]

$$(T_1E-T_10) > (T_2E-T_20),\ T_10 < T_20,\ T_1E > T_2E$$

In this embodiment, when nucleic acid-loaded lipid nanoparticles (LNPs) are produced, an amount of a rare component (e.g., a nucleic acid) can be minimized by shortening the inflow time of the rare component, for example. The inflow time can be calculated, for example, by the following expression:

[Mathematical 2]

$$T_10=W_1L/V_1,\ T_1E=(W_1L+W_1S)/V_1,\ T_20=W_2L/V_2,\ T_2E=(W_2L+W_2S)/V_2$$

A contact surface between a sample and a fluid flowing through the flow channel that occurs upon sample injection is preferably small in area (especially under a fast flow velocity in conditions for mass production). For example, when the sample is injected by valve switching, a time taken for switching flow channels by the valve is preferably short and, for example, the switching can be completed within about 1000 ms, within about 900 ms, within about 800 ms, within about 700 ms, within about 600 ms, within about 500 ms, within about 400 ms, within about 300 ms, within about 200 ms, within about 150 ms, within about 100 ms, within about 70 ms, or within about 50 ms. Since injection of the sample requires a period of time, albeit short, an injection time, as used herein, refers to a time point at which a sample begins to come into contact with a fluid flowing through a flow channel, unless otherwise limited. A time when the sample is injected may be controlled automatically by a programmable logic controller (PLC) or manually, for example.

Since a method of the present disclosure can form a composite by injecting a sample, various conditions can be examined by just changing a type and/or a concentration of a component in a sample to be injected. For example, composites prepared under different conditions can be easily obtained by preparing a plurality of samples in advance and sequentially transferring the samples to injection portions for flow channels by means of an autosampler. In one embodiment, a sample including a first component is injected into a middle of a first flow channel (a portion of the flow channel that is neither an inflow port nor an outflow port) and/or a sample including a second component is injected into a middle of a second flow channel.

In one embodiment, a first flow velocity and/or a second flow velocity is optimized for mass production and is fast for an amount of a sample to be injected. Typically, the sample is injected into a flow channel (flow channel in a liquid-feeding state) without stopping a flow of a first fluid and/or a second fluid. The flow of the fluid such as shear force can affect a property of a composite to be formed (e.g., particle diameter), so the sample is preferably injected without changing the flow velocity as much as possible in order to enable a scale-up under the same conditions. A time taken for switching valves is also preferably short from this viewpoint. In one embodiment, the first flow velocity and/or the second flow velocity can be each independently selected from about 1 mL/min, about 1.5 mL/min, about 2 mL/min, about 3 mL/min, about 4 mL/min, about 5 mL/min, about 6 mL/min, about 7 mL/min, about 8 mL/min, about 9 mL/min, about 10 mL/min, about 15 mL/min, about 20 mL/min, about 30 mL/min, about 40 mL/min, about 50 mL/min, about 60 mL/min, about 70 mL/min, about 80 mL/min, about 90 mL/min, about 100 mL/min, about 200 mL/min, about 300 mL/min, about 400 mL/min, about 500 mL/min, about 600 mL/min, about 700 mL/min, about 800 mL/min, about 900 mL/min, about 1000 mL/min, about 2000 mL/min, about 3000 mL/min, about 4000 mL/min, about 5000 mL/min, or a range between any two of these numerical values.

In one embodiment, amounts of samples to be injected into the first flow channel and/or the second flow channel at a time are each independently amounts that are finished flowing in a time (retention time) of about 50 ms, about 100 ms, about 150 ms, about 200 ms, about 300 ms, about 400 ms, about 500 ms, about 600 ms, about 700 ms, about 800 ms, about 900 ms, about 1000 ms, or a range between any two of these numerical values at the first flow velocity and/or the second flow velocity. In one embodiment, the amounts of the samples to be injected into the first flow channel and/or the second flow channel at a time can be each independently selected from about 50 μL, about 100 uL, about 150 μL, about 200 μL, about 300 μL, about 400 μL, about 500 μL, about 600 μL, about 700 μL, about 800 μL, about 900 μL, about 1000 μL, about 2000 μL, about 5000 μL, about 10000 μL, about 20000 μL, about 50000 μL, or a range between any two of these numerical values. In one embodiment, the amounts of the samples to be injected into the first flow channel and/or the second flow channel at a time can be each independently selected from about 50 μg, about 100 μg, about 150 μg, about 200 μg, about 300 μg, about 400 μg, about 500 μg, about 600 μg, about 700 μg, about 800 μg, about 900 μg, about 1000

μg, about 2000 μg, about 5000 μg, about 10000 μg, about 25000 μg, or a range between any two of these numerical values.

In one embodiment, in a step of injecting a sample including a component (e.g., a first component) into the flow channel, a retention time from when a sample injection portion is filled with the component until it is injected into a flow channel is independently within about 0.5 minutes, within about 1 minute, within about 1.5 minutes, within about 2 minutes, within about 3 minutes, within about 4 minutes, within about 5 minutes, within about 7 minutes, or within about 10 minutes. In one embodiment, a time from when a sample including a component (e.g., a first component) is injected into a flow channel (injection start time) until it reaches a combination portion is independently within about 0.1 seconds, within about 0.2 seconds, within about 0.5 seconds, within about 1 second, within about 1.5 seconds, within about 2 seconds, within about 3 seconds, within about 4 seconds, within about 5 seconds, within about 7 seconds, or within about 10 seconds.

In one embodiment, a fourth fluid flowing through a fifth flow channel may be combined with a third fluid at an additional combination portion to form a sixth flow channel through which a fifth fluid flows. A composite can be, for example, stabilized by adding the fourth fluid. An additional fluid may be combined as well. Flow velocities in the third flow channel and the sixth flow channel can usually be determined automatically from inner diameters of the first through sixth flow channels and the first, the second, and the fourth flow channels.

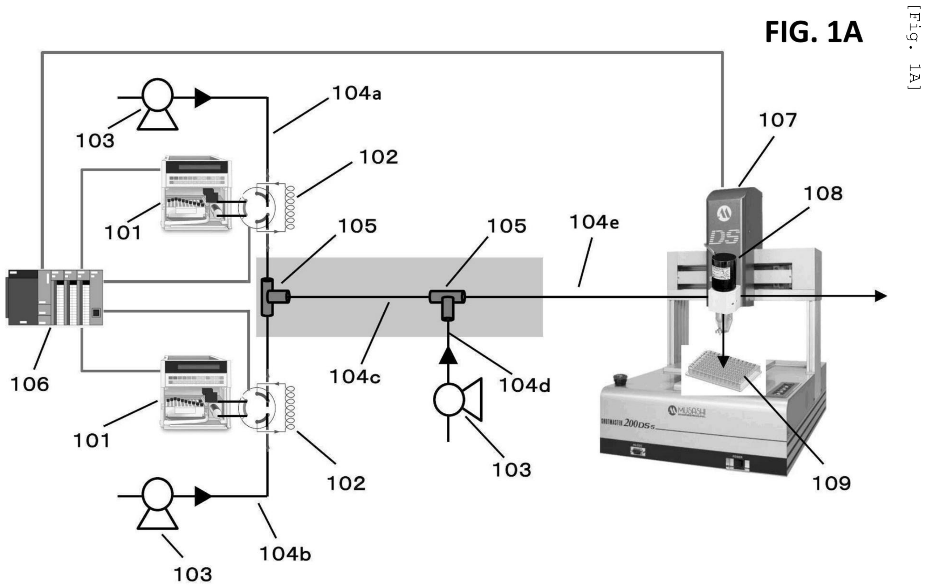

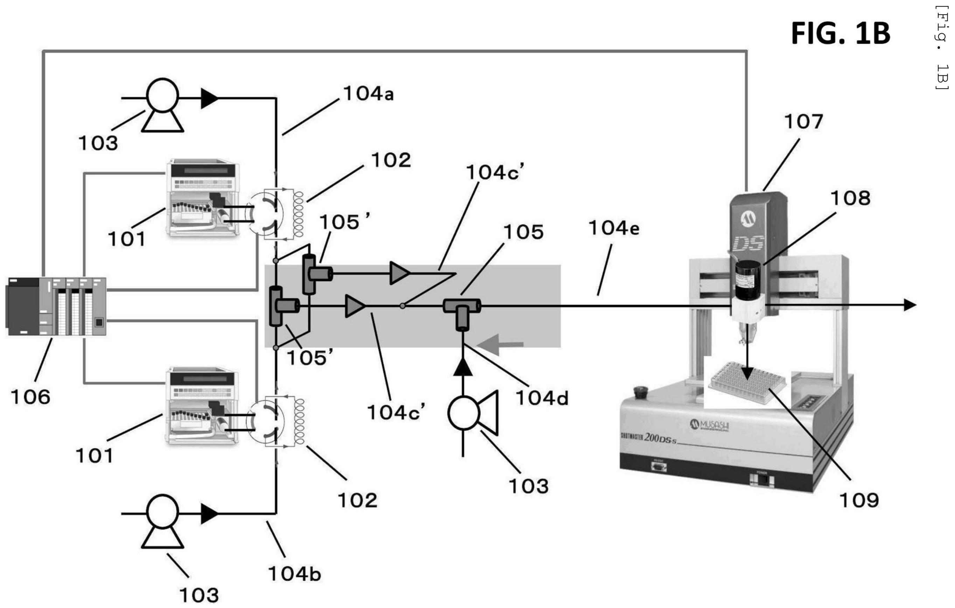

Types of conditions to be examined to form a composite in a method of the present disclosure (e.g., a flow velocity, a temperature, a type of component in a sample and its concentration) can be set by those skilled in the art as appropriate, and appropriate values of parameters can be determined by a method of the present disclosure. For example, when drug-loaded lipid particles are produced as shown in FIG. 1, a sample including a lipid (first component) in an alcohol (e.g., ethanol) is injected into a first flow channel through which an alcohol (first fluid) flows, and a sample including a drug (second component) such as a nucleic acid in water is injected into a second flow channel through which water (second fluid) flows. Primary dilution is made at a combination portion of the first flow channel and the second flow channel and the resulting primarily diluted solution is allowed to pass through a third flow channel over a predetermined time to reach a combination portion of the third flow channel and a fifth flow channel through which water flows. Then, secondary dilution is made and a composite can be collected from a sixth flow channel through which the resulting secondarily diluted solution flows. In this case, a particle size of lipid particles (e.g., average particle diameter: 10 to 1000 nm, 10 nm to 500 nm, 20 nm to 300 nm, 20 nm to 200 nm) can be controlled by an alcohol concentration in the primarily diluted solution, a lipid concentration in the sample, a drug concentration in the sample, the predetermined time, a Reynolds number (Nre) in the third flow channel, pressure, a temperature during mixing, etc.

In one embodiment, an alcohol concentration in the primarily diluted solution is preferably in a range where a liposome membrane is not sufficiently stabilized, and an alcohol concentration in the secondarily diluted solution is preferably in a range where the liposome membrane is stabilized. In one embodiment, the alcohol concentration in the primarily diluted solution can be 10 to 50% by weight, e.g., about 10% by weight, about 15% by weight, about 18% by weight, about 20% by weight, about 25% by weight, about 30% by weight, about 35% by weight, about 40% by weight, about 45% by weight, or about 50% by weight. In one embodiment, the alcohol concentration in the secondarily diluted solution can be 0 to 30% by weight, e.g., about 0% by weight, about 5% by weight or less, about 10% by weight or less, about 15% by weight or less, about 18% by weight or less, about 20% by weight or less, about 25% by weight or less, or about 30% by weight or less. In one embodiment, the predetermined time for the primarily diluted solution to pass through the third flow channel (which can be controlled by at least one of a length and a flow velocity of the third flow channel) may be about 0.1 seconds to 60 minutes, for example, about 0.1 seconds, about 0.2 seconds, about 0.5 seconds, about 1 second, about 2 seconds, about 5 seconds, about 10 seconds, about 30 seconds, about 1 minute, about 2 minutes, about 5 minutes, about 10 minutes, about 15 minutes, about 20 minutes, about 25 minutes, about 30 minutes, about 45 minutes, about 60 minutes, or a range between any two of these numerical values, or may also be further longer. In one embodiment, the Reynolds number (Nre) in the third flow channel can be less than 2000, less than 1000, less than 500, less than 300, less than 200, less than 100, less than 50. In one embodiment, the pressure in the third flow channel can be 0.5 MPa or more, 0.8 MPa or more, 1 MPa or more, 1.5 MPa or more, 2 MPa or more, 3 MPa or more, 4 MPa or more, 5 MPa or more, 8 MPa or more, or 10 MPa or more. In one embodiment, a surface of a lipid particle may be modified with a modifying agent. Each sample may include an additive such as an osmoregulating agent, a stabilizing agent, an antioxidizing agent, a pH adjusting agent.

In one embodiment, the method may further include a step of detecting a combination state of a first component and a second component downstream of a combination portion (e.g., on a third flow channel, a fifth flow channel). The combination state includes a state selected from the group consisting of a concentration of at least one of the first component and the second component, a particle diameter of particles in a flow, and a state of a chemical bond between the first component and the second component. Such a detection can be performed using a photodiode array (PDA) detector, an ultraviolet (UV) detector, a differential refractive index (RI) detector, a fluorescence detector, a circular dichroism detector, an optical rotation detector, etc.

In one embodiment, the method may further include a step of removing a fraction including a composite of a first component and a second component from a collection portion of a third flow channel or a sixth flow channel at a third time.

In one embodiment, the collection portion is positioned in a middle of the third flow channel or the sixth flow channel. In one embodiment, the method may further include a step of dispensing the fraction removed from the collection portion to a receiving portion. In one embodiment, the dispensing step includes dispensing a plurality of fractions to a receiving portion containing a plurality of compartments. In one embodiment, the method may include a step of testing a plurality of fractions. In one embodiment, a time when the fraction is removed from the collection portion is controlled based on a time when the component is injected into the flow channel.

In one embodiment, the present disclosure provides a method for screening a condition for producing drug-loaded lipid particles. The method may include a step of preparing a series of flow channels including a first flow channel through which a first fluid flows at a first flow velocity, a second flow channel through which a second fluid flows at a second flow velocity, and a third flow channel through which a third fluid flows at a third flow velocity, the third flow channel being formed by combining the first flow channel and the second flow channel at a combination portion; (1) a step of injecting a sample including a candidate lipid into the first flow channel at a first time; (2) a step of injecting a sample including a candidate drug into the second flow channel at a second time; (3) a step of removing a fraction including particles of the candidate lipid from a collection portion of the third flow channel at a third time; and evaluating the particles of the candidate lipid. The first time and the second time may be set so that the candidate lipid and the candidate drug simultaneously reach the combination portion. In one embodiment, the method further includes a step of preparing a plurality of the particles of the candidate lipid, comparing the plurality of the particles of the candidate lipid, and selecting hit lipid particles by repeating (1) to (3). In one embodiment, a method for producing hit lipid particles under conditions for the flow channel and the flow velocity set in such a method for screening is also provided herein. In one embodiment, a frequency with which (1) to (3) are performed can be increased, thereby increasing an amount of the hit lipid particles to be produced.

(Apparatus and system)

In one aspect, the present disclosure provides an apparatus or a system for injecting a plurality of samples into a plurality of flow channels at a time when a first component and a second component are mixed and the apparatus or the system can be used in a method described herein. The apparatus or system can include a first flow channel through which a first fluid flows; a first injection portion from which a sample including a first component is injected into the first flow channel; a second flow channel through which a second fluid flows; a second injection portion from which a sample including a second component is injected into the second flow channel; a combination portion in which the first flow channel and the second flow channel combine with each other; a third flow channel through which a third fluid flows and which extends from the combination portion; and a control portion. The control portion may be configured to control a time when the first injection portion injects the first component into the first flow channel and a time when the second injection portion injects the second component into the second flow channel. In one embodiment, the apparatus or the system includes a fourth flow channel through which a third component flows and a third injection portion that injects the third component into the fourth flow channel, the control portion may be configured to control a time when the first injection portion injects the first component into the first flow channel, a time when the second injection portion injects the second component into the second flow channel, and a time when the third injection portion injects the third component into the fourth flow channel, and an injection portion for injecting more components as well may be provided. Such an operation and feature of the fourth flow channel and the injection portion are understood in the same manner as for the first flow channel, the second flow channel, and the injection portion.

In one embodiment, the first injection portion is positioned in a middle of the first flow channel and the second injection portion is positioned in a middle of the second flow channel. In one embodiment, the apparatus or the system further includes a fifth flow channel through which a fourth fluid flows, an additional combination portion in which the fifth flow channel and the third flow channel combine with each other, a sixth flow channel through which a fifth fluid flows and which extends from the additional combination portion.

In the apparatus or the system of the present disclosure, the first injection portion and/or the second injection portion can include a valve. In one embodiment, the valve may be each independently a valve including a sample injection pipe (e.g., sample room) having a volume of about 0.05 mL or less, about 0.1 mL or less, about 0.15 mL or less, about 0.2 mL or less, about 0.3 mL or less, about 0.4 mL or less, about 0.5 mL or less, about 0.6 mL or less, about 0.7 mL or less, about 0.8 mL or less, about 0.9 mL or less, about 1 mL or less, about 2 mL or less, about 3 mL or less, about 4 mL or less, about 5 mL or less, about 7 mL or less, about 10 mL or less, about 20 mL or less, about 30 mL or less, about 40 mL or less, about 50 mL or less, about 100 mL or less, about 200 mL or less, about 300 mL or less, about 400 mL or less, or about 500 mL or less. In one embodiment, the valve is each independently a valve capable of injecting the first component and/or the second component into the first flow channel and/or the second flow channel within about 0.05 seconds, within about 0.1 seconds, within about 0.15 seconds, within about 0.2 seconds, within about 0.3 seconds, within about 0.4 seconds, within about 0.5 seconds, within about 0.6 seconds, within about 0.7 seconds, within about 0.8 seconds, within about 0.9 seconds, or within about 1 second.

In the apparatus or the system of this disclosure, a pump (a portion with a variable flow velocity) can be attached at any position. The pump can be, for example, a syringe pump, a plunger pump, a piston pump, or a roller pump. The pump can adjust a flow velocity, pressure, etc. In one embodiment, the pump through which the first fluid and/or the second fluid flows is a plunger pump. In the present disclosure, the plunger pump may be suitable since it may be preferred to maintain a constant flow velocity for a long period of time for examination of various conditions. In one embodiment, the pump in the apparatus or the system of the present disclosure can provide a flow velocity of about 2 mL/min or more, about 5 mL/min or more, about 10 mL/min or more, about 20 mL/min or more, about 30 mL/min or more, about 40 mL/min or more, about 50 mL/min or more, about 60 mL/min or more, about 70 mL/min or more, about 80 mL/min or more, about 90 mL/min or more, about 100 mL/min or more, about 200 mL/min or more, about 300 mL/min or more, about 400 mL/min or more, about 500 mL/min or more, about 600 mL/min or more, about 700 mL/min or more, about 800 mL/min or more, about 900 mL/min or more, about 1000 mL/min or more, about 2000 mL/min or more, about 3000 mL/min or more, about 4000 mL/min or more, or about 5000 mL/min or more. For many research pumps, such a high flow velocity cannot be set. In one embodiment, a back pressure on the pump through which the first fluid and/or the second fluid flows is about 0.2 MPa or more, about 0.5 MPa or more, about 1 MPa or more, about 1.5 MPa or more, about 2 MPa or more, about 3 MPa or more, about 4 MPa or more, about 5 MPa or more, about 6 MPa or more, about 7 MPa or more, about 8 MPa or more, about 9 MPa or more, about 10 MPa or more, about 15 MPa or more, about 20 MPa or more, about 25 MPa or more, about 30 MPa or more, about 35 MPa or more, about 40 MPa or more, about 45 MPa or more, or about 50 MPa or more. In one embodiment, the pump in the apparatus or the system of the present disclosure is capable of withstanding such a high flow rate and a high pressure and a pump for high-performance liquid chromatography may be utilized. In the present disclosure, a fluid does not need to flow through a narrow flow channel with high resistance or a flow channel including a filler, but the fluid can be assumed to flow through a long flow channel at a high flow velocity, which can increase the back pressure on the pump. The present application makes the first attempt to use such a high flow velocity and/or high pressure-resistant pump to inject or collect a small amount of a sample (e.g., 1 mL or less of a sample or 1 mg or less of a component), and configuration of a method, a system, or an apparatus of the present disclosure for this purpose has not previously been available. Since some desired lipid particles may not be successfully obtained unless the particles are prepared at a high back pressure, the method, the system, or the apparatus of the present disclosure may be suitable for preparation of such lipid particles.

In the apparatus or the system of the present disclosure, any tube can be used for feeding a liquid and a material of the tube can be determined, for example, taking thermal insulation (thermal conductivity), heat resistance, chemical resistance, or a sealing property into consideration. Examples of a material for a liquid-feeding tube (which is also a flow channel wall) and/or a connecting portion to form a flow channel include, but not limited to, glass, a thermoplastic (e.g., polyvinyl chloride with a plasticizer), a thermoplastic elastomer (e.g., polyvinyl chloride without a plasticizer; a polypropylene-based plastic including a copolymer of styrene-ethylene-butylene and a silicone oil or USP petroleum; polyether ether ketone (PEEK) (aromatic polyetherketone to which a benzene ring is attached by an ether group and a ketone group), a thermosetting rubber (e.g., a siloxane polymer with non-crystalline silica), or a thermocoagulable fluororubber. The flow channel may have any inner diameter, e.g., about 0.1 mm, about 0.15 mm, about 0.2 mm, about 0.3 mm, about 0.4 mm, about 0.5 mm, about 0.6 mm, about 0.7 mm, about 0.8 mm, about 0.9 mm, or about 1 mm.

In the apparatus or the system of the present disclosure, a combination portion may include a mixer. The mixer can be a T-mixer, a mixer including a microflow channel apparatus, or a static mixer, and an appropriate mixer can be selected considering a factor such as a composition, a flow velocity, and pressure of a solution. The mixer may be installed downstream of the combination portion (e.g., static mixer).

In one embodiment, each of the first flow channel and the second flow channel includes a plurality of branched flow channels, and the combination portion includes a plurality of branched flow combination portions at which branched flow channels from the first flow channel and branched flow channels from the second flow channel are combined. In one embodiment, the first flow channel and/or the second flow channel is branched to form the branched flow channel. In one embodiment, the branched flow channels downstream of the branched flow combination portion combine with each other in the third flow channel. Use of such a branched flow channel allows for a scale-up while maintaining the same conditions.

In one embodiment, the third flow channel or the sixth flow channel includes a collection portion, and a collection apparatus is attached to the collection portion. In one embodiment, the control portion further controls a time of collection by the collection device. In one embodiment, the collection portion is positioned in a middle of the third flow channel or the sixth flow channel. In one embodiment, the collection device includes a valve. In one embodiment, the valve is a slide solenoid valve. In one embodiment, the apparatus or the system further includes a receiving portion that receives a fraction removed from the collection device. In one embodiment, the receiving portion includes a plurality of compartments that receive a plurality of fractions. In one embodiment, the apparatus or the system includes a detector downstream of the combination portion.

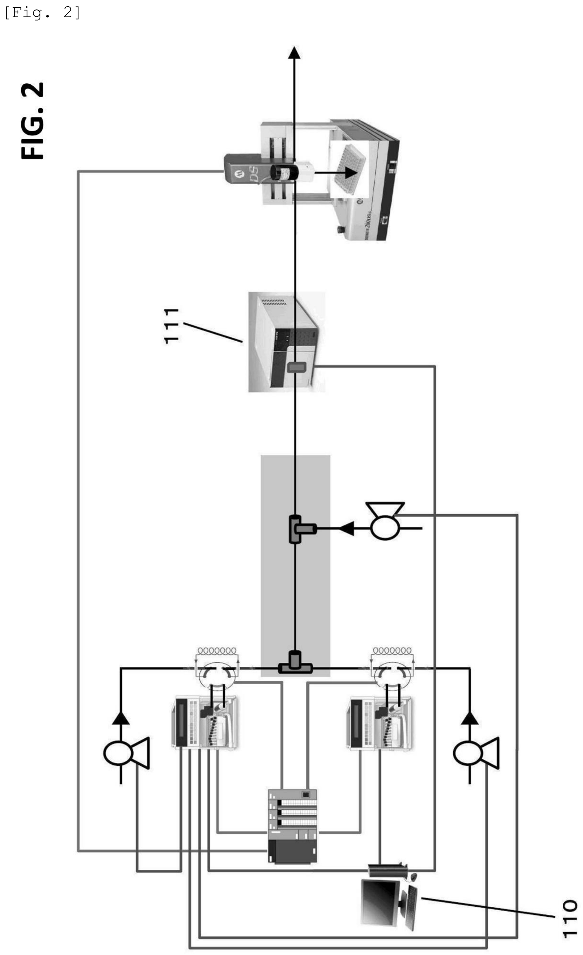

An apparatus or a system of the present disclosure is described with reference to specific embodiments shown in FIGs. 1 to 3. As shown in FIG. 1A, the apparatus or the system of the present disclosure includes at least a first flow channel (104a) and a second flow channel (104b), each of which includes an injection portion (102). Fluids can flow through pumps into the first flow channel (104a) and the second flow channel (104b), respectively, and flow velocities thereof can be each independently set. An autosampler (101) is attached to the injection portion (102) to automatically form a composite under various conditions. The first flow channel (104a) and the second flow channel (104b) are connected to a combination portion (105), from which a third flow channel (104c) extends. After a first component and a second component are mixed together, a fifth flow channel (104d) may be further combined at an additional combination portion (105), in which case the composite flows through a sixth flow channel (104e). After the first component and the second component are mixed together, a fourth flow channel (which may be 104d) through which a third component flows may be further combined at the additional combination portion (105). If only a specific fraction is desired to be fractionated, the fraction is removed by a collection device (107) (which may include a valve (108)) attached to the third flow channel or the sixth flow channel and collected in a receiving portion (109). A control portion (106) controls the injection portions (102) on at least the first flow channel (104a) and the second flow channel (104b) and gives an instruction regarding a time when a sample is injected so that the first component and the second component are present simultaneously and can contact (mix) with each other at the combination portion (105) where these flow channels are combined. The control portion (106) may control the autosampler (101) to automatically fill the injection portion (102) with (different) samples in sequence. The control portion (106) may control the collection device (107) to collect only a specific fraction (including the composite) at a specific timing. As shown in FIG. 2, in the apparatus or the system of the present disclosure, a detector may be installed on the third flow channel or the sixth flow channel downstream of the combination portion or the additional combination portion. The detector can detect the first component, the second component, and/or the composite. The apparatus or the system of the present disclosure may further include a control portion (computer, 110) that controls a fluid (e.g., flow velocity) flowing through the flow channel and thus can simultaneously control the autosampler and the detector.

As shown in FIG. 1B, the apparatus or the system of the present disclosure can also be configured to include a branched flow channel (104c') and/or a branched flow combination portion (105').

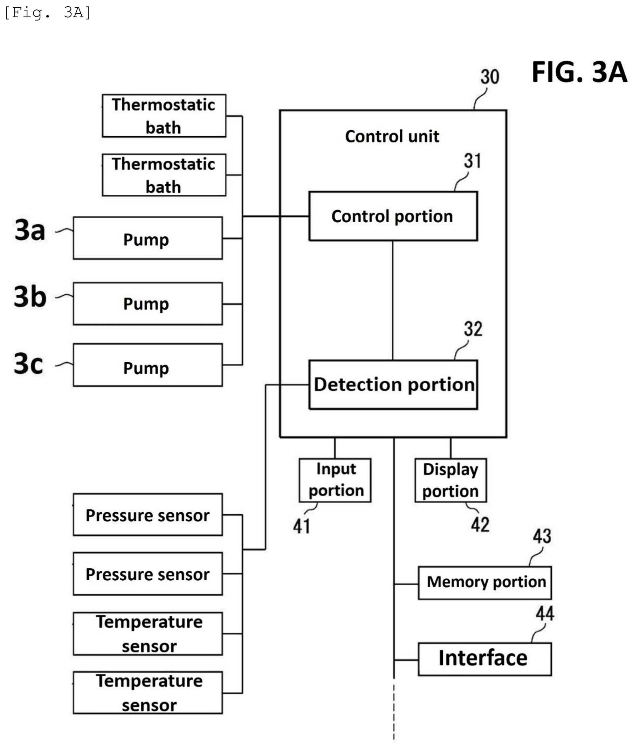

The system of the present disclosure (lipid particle production system) can have a control unit 30 as shown in FIG. 3A. The control unit 30 includes a control portion 31 and a detection portion 32. The control portion 31 and the detection portion 32 are communicatively connected to each other. The above-described control may be made possible by hardware (e.g., a dedicated circuitry) alone, or the above-described control may be performed by causing a CPU to execute a program.

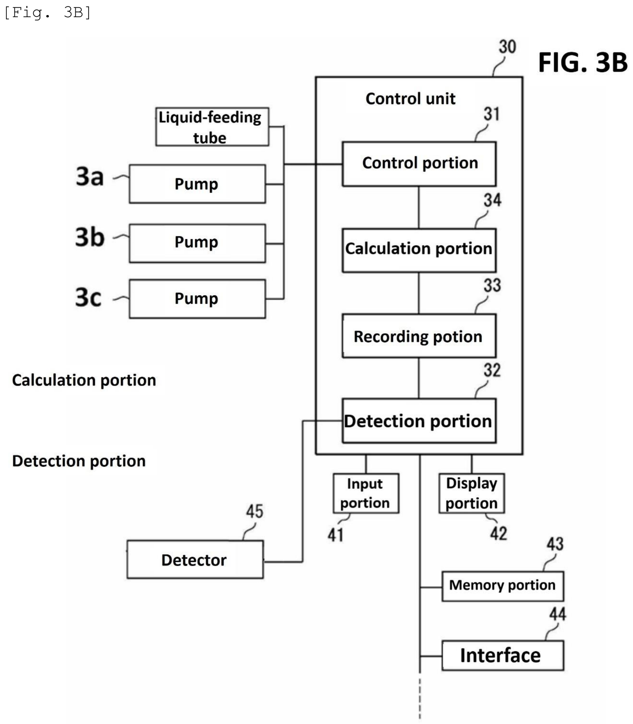

In one aspect, the system of the present disclosure (lipid particle production system) can have a control unit 30 as shown in FIG. 3B. The control unit 30 includes a control portion 31, a detection portion 32, a recording portion 33, and a calculation portion 34. The control portion 31, the detection portion 32, the recording portion 33, and the calculation portion 34 are communicatively connected to each other. The above-described control may be made possible by hardware (e.g., a dedicated circuitry) alone, or the above-described control may be performed by causing a CPU to execute a program.

A detector 45 detects a concentration of a component or a particle size of lipid particles to be produced. A datum acquired by the detector 45 is transmitted to the detection portion 32 and stored in the recording portion 33. The calculation portion 34 calculates operating conditions of the system such as a flow velocity to be evaluated next, based on information stored in the recording portion 33 (along with an input from an input portion 41, if necessary).

The control portion 31 is composed of a central processing unit (CPU), a read-only memory (ROM), a random access memory (RAM), and a driving circuit for various actuators included in a liposome production apparatus. The ROM stores various programs such as a basic input/output system (BIOS), an operating system (OS), various drivers, and various applications. The detection portion 32 is composed of a detection circuit for various sensors (e.g., a temperature sensor, a pressure sensor, and a detector) included in the liposome production apparatus.

The control unit 30 is communicatively connected to each of an input portion 41, a display portion 42, a memory portion 43, and an interface 44. The interface 44 allows a datum to be sent and received between the control unit 30 and an external apparatus. The control unit 30 is connected to a general-purpose computer (so-called personal computer), for example, via the interface 44.

The input portion 41 receives an input from a user. The input portion 41 is composed of, for example, a keyboard, a mouse, or a touch panel. The display portion 42 is composed of, for example, a display such as a liquid crystal display (LCD) or an electroluminescent display (ELD). Note that, when the input portion 41 and the display portion 42 are composed of a touch panel, the input portion 41 and the display portion 42 are integrated.

The memory portion 43 is composed of a nonvolatile memory such as a hard disk. The memory portion 43 stores a program and a datum pertaining to various controls (e.g., a datum input from the input portion 41 to the control unit 30). The control unit 31 controls a liquid-feeding tube and pumps 3a, 3b, and 3c based on information output from the calculation portion 34.

The control portion 31 controls at least one of a thermostatic bath, a liquid-feeding tube, pump 3a, 3b and 3c based on at least one of a datum input from the input portion 41 to the control portion 30, a signal output from each of a temperature sensor and a pressure sensor input to the detection portion 32, and information output from the calculation portion 34. A length of the liquid-feeding tube can be controlled, for example, by switching flow channels.

(Program)

In one aspect, the present disclosure provides a program for causing an apparatus including a processor to implement a method for injecting a plurality of components into a plurality of flow channels connected to a combination portion, which may be used in a method, an apparatus, or a system described herein. The method to be implemented by the program can cause a processor to implement a processing including receiving information about a first flow channel and information about a second flow channel; determining a first time at which a first component is injected into the first flow channel and a second time at which a second component is injected into the second flow channel based on the information about the first flow channel and the information about the second flow channel, the first time and the second time being times set so that the first component injected into the first flow channel and the second component injected into the second flow channel reach a combination portion at a time when the first component and the second component are mixed; instructing an injection device to inject the first component into the first flow channel at the first time; and instructing an injection device to inject the second component into the second flow channel at the second time. In one embodiment, the processing further includes receiving information about a third flow channel downstream of the combination portion; determining a third time at which a fraction is removed from the third flow channel based on the information about the third flow channel; and instructing a collection device to remove the fraction from the third flow channel at the third time.

Any control by the control portion described herein may be performed by a method coded by the program. In one aspect, the present disclosure also provides a recording medium in which the program described herein is recorded or stored.

(Formulation)

The present disclosure also provides a composite produced by a method of the present disclosure or a formulation including the composite. The composite produced by the method of the present disclosure or the formulation including the composite can be used for any application, for example, a pharmaceutical application, food, cosmetics, agriculture, imaging, etc., as well as an insecticide, a herbicide, a cosmetic agent, a fragrance, a food additive, a flavoring agent, an imaging agent, a dye, a fluorescent marker, a hair growth agent, a moisturizing agent, a dyestuff, a whitening agent, a pigment, an X-ray contrast agent, a ultrasound diagnostic agent, a radioisotope-labeled nuclear medicine diagnostic agent, a nuclear magnetic resonant diagnostic agent, etc. The formulation may include an additive such as an osmoregulating agent, a stabilizing agent, an antioxidizing agent, and a pH adjusting agent.

The stabilizing agent is not particularly limited, and examples thereof include a sugar such as glycerol, mannitol, sorbitol, lactose, or sucrose, and a sterol such as cholesterol.

The antioxidizing agent is not particularly limited, and examples thereof include ascorbic acid, uric acid, a tocopherol homologue (e.g., vitamin E). Note that, the tocopherol includes four isomers: $\alpha$, $\beta$, $\gamma$, and $\delta$, all of which can be used in the present invention.

The pH adjusting agent may be any basic or acidic compound, such as sodium hydroxide, citric acid, acetic acid, triethanolamine, sodium hydrogen phosphate, sodium dihydrogen phosphate, sodium malate, sodium succinate, etc.

Examples of the other additive include a pharmaceutically acceptable organic solvent, collagen, polyvinyl alcohol, polyvinyl pyrrolidone, a carboxyvinyl polymer, sodium carboxymethyl cellulose, sodium polyacrylate, sodium alginate, water soluble dextran, sodium carboxymethyl starch, pectin, methylcellulose, ethylcellulose, xanthan gum, gum arabic, casein, gelatin, agar, diglycerin, propylene glycol, polyethylene glycol, vaseline, paraffin, stearyl alcohol, stearic acid, human serum albumin (HSA), PBS, a biodegradable polymer, a serum-free medium, a surfactant acceptable as a pharmaceutical additive, and a buffer at a physiological pH.

(General tecnologies)

The analytical, chemical, and pharmaceutical methods used herein are well known and customary in the art and are described, for example, in (Gregory Gregoriadis, Liposome Technology: Liposome Preparation and Related Techniques, September 12, 2006 CRC Press, ISBN 9780849388217), which is incorporated herein by reference in its relevant part (possibly in its entirety).

As used herein, the term "or" is used when "at least one or more" of matters enumerated in a text can be employed. The same is true for "alternatively". When the phrase "within a range" of "two values" is specified herein, the range includes the two values themselves.

References cited herein such as the scientific literature, patents, and patent applications are incorporated herein by reference in their entirety as if specifically set forth herein.

The present disclosure has been described with reference to preferred embodiments for ease of understanding. Hereinafter, the present disclosure will be described with reference to Examples, but the above description and Examples below are provided for illustrative purposes only and are not intended to limit the present disclosure. Accordingly, the scope of the present disclosure is not limited to embodiments or Examples specifically described herein, but is limited only by claims.

[Example]

(Example 1: Construction of system)

A system was constructed as shown in FIG. 1A. An autosampler (101) and a high-speed automated valve (102) were controlled by a PLC (106) so that a nucleic acid (flowed through a flow channel 104a) and a lipid (flowed through a flow channel 104b) were mixed in a mixer (105). A high-speed dispenser (107) was used to precisely fractionate a small amount of lipid particles flowing at a high velocity. A solenoid slider valve (108) was attached to the high-speed dispenser to prevent the small amount of lipid particles from being agitated or diluted in a valve. The PLC also controlled the high-speed dispenser (107), which fully automated from injection of the nucleic acid and the lipid to collection of the lipid particles.

As shown in FIG. 2, an in-line detector (111) was further attached to quantify a concentration of the nucleic acid (lipid) in real time. An LC system was controlled by a computer (which could also be a control portion) (110). An HPLC apparatus (a controller portion therein) and the PLC were directly communicated with each other. A computer ran an HPLC control application, and signals were exchanged by the HPLC apparatus itself. For example, the following operation was possible: 1) a signal was output to the PLC when the autosampler 1 is ready, 2) a signal was output to the PLC when the autosampler 2 is ready, and 3) a time program start signal was output to the HPLC apparatus at a time when both the signals from 1) and 2) were output.

(Example 2: Examination of mixing timing)

In a method and a system of the present disclosure, different samples need to be timely mixed, and conditions for achieving this purpose were examined. Mixing of a nucleic acid sample (detected by absorbance at OD 260) and a lipid sample (detected by absorbance at OD 600), which were distinguishable from each other due to different absorbance wavelengths, was tested. The samples were injected into separate flow channels and combined with each other, from which consecutive fractions were obtained and then absorbance of each fraction was measured.

A valve was attached to a flow channel with a flow velocity of 45 mL/min, from which 100 μL of the nucleic acid sample was injected, and a valve was attached to another flow channel with a flow velocity of 15 mL/min, from which 200 μL of the lipid sample was injected. These flow channels were combined at a mixer. Mixing was tested by changing a pipe from the valve to the mixer under conditions where the two valves were simultaneously signaled to inject the sample.

The results are shown in Table 4. When the two flow channels were fitted with pipes having the same pipe inner diameter and pipe length, a difference in flow velocity between the flow channels caused the samples to enter the mixer at different times, resulting in maximum concentrations of the nucleic acid and the lipid in different fractions (FIG. 4A). Next, when a pipe with an inner diameter of 0.381 mm and a length of 76 cm (87 μL) was added to the flow channel into which the nucleic acid sample was injected, the fraction including the maximum concentration of the nucleic acid approached that of the lipid (FIG. 4B). Furthermore, when the pipe added to the flow channel into which the nucleic acid sample was injected was changed to a pipe with an inner diameter of 0.381 mm and a length of 134 cm (153 μL), the nucleic acid was included at the maximum concentration in the fraction with a sufficiently high concentration of the lipid (FIG. 4C). When the pipe from the valve to the mixer in the flow channel into which the lipid sample was injected was changed to a smaller volume (-10 μL) pipe with an inner diameter of 0.18 mm and a length of 6 cm under the condition under which the pipe with an inner diameter of 0.381 mm and a length of 76 cm (87 μL) was added to the flow channel into which the nucleic acid sample was injected, the nucleic acid was included at the maximum concentration in the fraction with a sufficiently high concentration of the lipid under this condition as well (FIG. 4D).

Next, we attempted to properly mix the two components by controlling a timing of injection, with pipes having the same pipe inner diameter and pipe length being installed in a region from the valve to the mixer for the two flow channels. A high-speed automatic valve (VF-02: FLOM Corporation) was used as the valve.

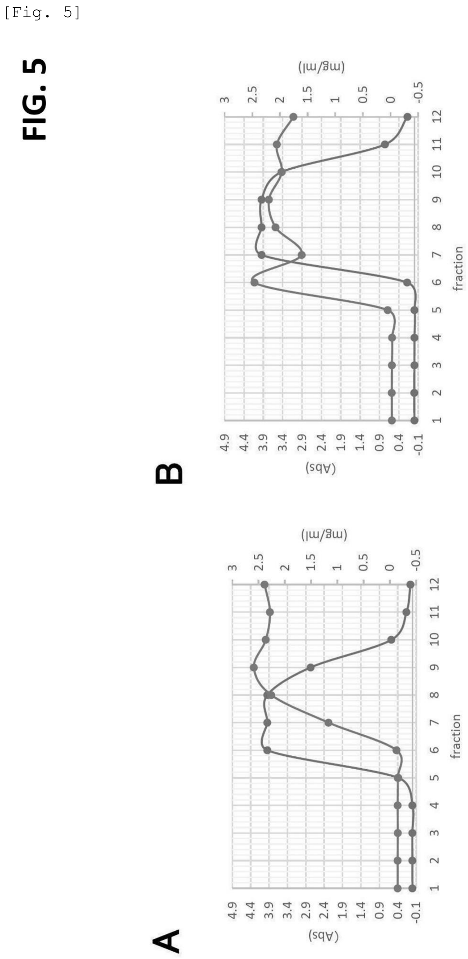

The results are shown in Table 5. When the two valves were simultaneously signaled to inject the sample, a difference in flow velocity between the flow channels caused the samples to enter the mixer at different times, resulting in maximum concentrations of the nucleic acid and the lipid in different fractions (FIG. 5A). Next, when the time when the nucleic acid sample was injected was delayed by 100 msec from the time when the lipid sample was injected, the nucleic acid was included at the maximum concentration in the fraction with a sufficiently high concentration of the lipid (FIG. 5B). Note that, a lower lipid concentration in the seventh fraction is expected to result from an experimental error caused by the fluid in the flow channel for the lipid pushed back due to pressure fluctuation caused by a delay of a valve switching operation.

Such an examination can easily set conditions for achieving appropriate mixing between components.

(Example 3: Examination of valve)

For the apparatus configuration of Example 1, an effect of the valve on injection of a sample and collection of lipid particles was investigated.

Conditions used in this test were as follows.

· Nucleic acid sample: nucleic acid 1.28 mg/mL (in water), injection volume 200 µL.
·Lipid sample: lipid composition of DOTAP:DPPC:Cholesterol:DSPE-MPEG2K = 50:19.5:30:0.5, lipid concentration: 50 mg/mL (in ethanol), injection volume 200 µL.
·Flow velocity (nucleic acid flow channel: 45 mL/min, lipid flow channel: 15 mL/min), N/P ratio = 3.0
·Pipe from injection portion for nucleic acid sample to mixer: inner diameter 0.381 mm, length 76 cm
·Pipe from injection portion for lipid sample to mixer: inner diameter 0.18 mm, length 6 cm
·Flow velocity of flow channel for water for secondary dilution: 23.3 mL/min.
·Internal diameter of flow channel after secondary dilution: 0.5 mm.
· Liquid volume of fractions obtained: 80 µL per fraction.
· Time interval to acquire next fraction: 0 ms (This was a set value on the apparatus. In fact, some time interval was considered to have occurred).
· Autosampler settings (HPLC control application set up on PC): input items; sample number, velocities of sample drawn or injected, injection volume (sample volume + excess volume), air injection volume before and after sample, needle + injection port cleaning settings before and after injection, request signal output before sample injection, sample injection start signal input.

An autosampler, a valve, and a dispenser (collection device) were controlled according to a program as follows.

1) Send an injection-ready signal to the PLC from each of two autosamplers (master and slave).
2) Send a time program start signal from a PLC to the autosamplers at a time when the injection-ready signals were sent from the two autosamplers (a time program on a master side was used).
3) Start the pump by the time program on the master side.
4) Send a program start signal for a dispenser from the autosampler to the PLC by the time program.
5) Send a program start signal from the PLC to the dispenser.
6) Start a program for the dispenser (after cleaning a fraction port, move the dispenser to a fractionation start position (standby state for a fractionation start signal)).
7) After the pump reaches a set flow rate (time setting), send an injection request signal from the autosampler (master side) to the PLC.
8) Send injection signals from the PLC to the two valves. At the same time as the injection signals, send a signal to start fractionation and a signal to switch from a waste liquid side to a fractionation side to the dispenser.
9) Fractionate a sample by the dispenser (a number and a volume of fraction per well of a 96-well plate were pre-set in the program for the dispenser).
10) After the fractionation, move the dispenser to a cleaning position, clean a flow channel, and then move it to the next fractionation starting position.

The following three valve conditions were tested:

·0.8s + DV: low-speed automatic valve (FCV-12AH: SHIMADZU CORPORATION) + diaphragm valve (MTV-3-1: Takasago Electric, Inc.)
·0.1s + DV: high-speed automatic valve (VF-02: FLOM Corporation) + diaphragm valve
· 0.1s + SV: high-speed automatic valve + slider valve (MTV-3SL: Takasago Electric, Inc.)

Fractions acquired under each condition were examined for a nucleic acid concentration (detected by absorbance at OD 260), a lipid concentration (detected by absorbance at OD 600), and a particle size distribution of lipid particles (measured by a dynamic light scattering method).

(Results)

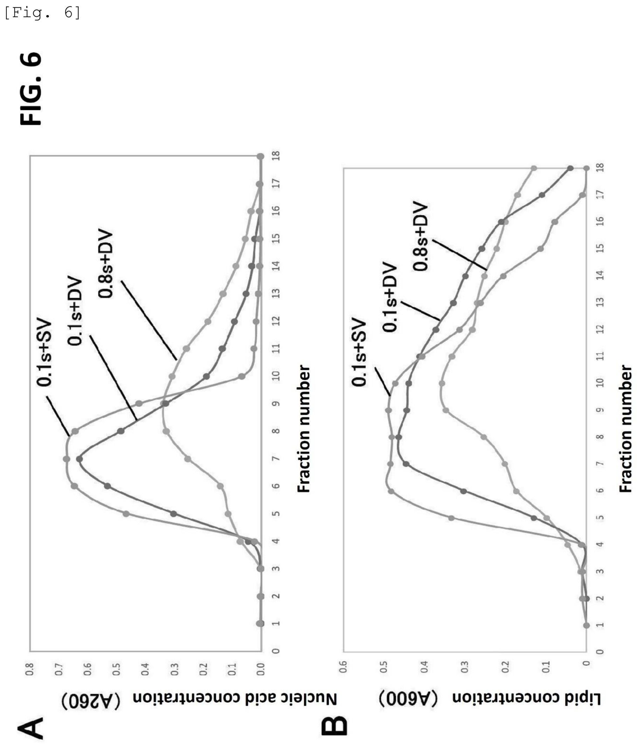

Regarding the nucleic acid concentration (FIG. 6A), for 0.1s + SV, there were only a small number of fractions with intermediate concentrations before reaching a peak concentration and the peak concentration was plateaued over three fractions, suggesting that the same effect was obtained in these fractions as when a nucleic acid having the same concentration as an injected sample was kept flowing. For 0.1s + DV, it was observed that a peak concentration was slightly lower than that for 0.1s + SV, the number of fractions with intermediate concentrations below the peak concentration was increased, and more fractions did not adequately reflect a nucleic acid concentration in the injected sample due to dilution. For 0.8s + DV, it was observed that a peak concentration was lower and the nucleic acid concentration was lower than that of the injected sample as a whole due to dilution.

Regarding the lipid concentration (FIG. 6B), for 0.1s + SV, there were only a small number of fractions with intermediate concentrations before reaching a peak concentration and the peak concentration was plateaued over five fractions, suggesting that the same effect was obtained in these fractions as when a lipid having the same concentration as an injected sample was kept flowing. For 0.1s + DV, it was observed that a peak concentration was slightly lower than that for 0.1s + SV, the number of fractions with intermediate concentrations below the peak concentration was increased, and more fractions did not adequately reflect a lipid concentration in the injected sample due to dilution. For 0.8s + DV, it was observed that a peak concentration was lower and the lipid concentration was lower than that of the injected sample as a whole due to dilution.

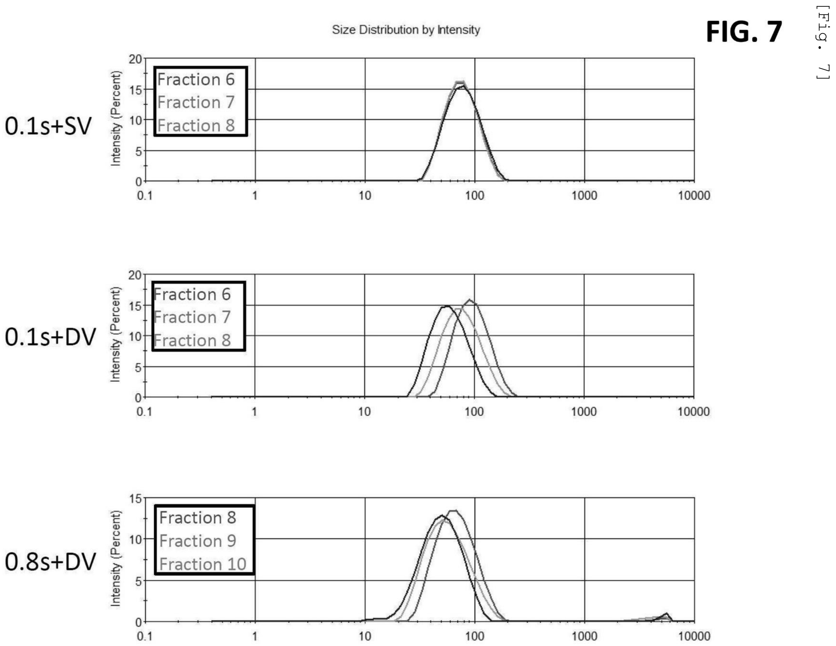

For a particle size distribution (FIG. 7 and the table below), average particle diameters of the three fractions that gave the peak concentration of the nucleic acid were nearly identical to each other. For 0.1s + DV, the average particle diameter of the fraction that gave the peak concentration of the nucleic acid was different from those of the fractions therearound, possibly due to a nonconstant ratio of a nucleic acid to lipid (A260/A600). For 0.8s + DV, the three fractions that gave the highest peak concentration of the nucleic acid had a small average particle diameter, probably because a peak concentration of the nucleic acid was low due to dilution, and also had a large PDI and coarse particles due to non-uniform mixing.

[Table 1]

|  | Fraction number | A260/A600 | Z-average(nm) | PDI |
|---|---|---|---|---|
|  | 6 | 1.34 | 71.06 | 0.110 |
| 0.1s+SV | 7 | 1.39 | 70.08 | 0.122 |
|  | 8 | 1.34 | 72.04 | 0.114 |
|  | 6 | 1.75 | 90.26 | 0.130 |
| 0.1s+DV | 7 | 1.41 | 70.80 | 0.140 |
|  | 8 | 1.05 | 55.86 | 0.150 |
|  | 8 | 1.29 | 62.92 | 0.167 |
| 0.8S+DV | 9 | 0.98 | 52.57 | 0.195 |
|  | 10 | 0.86 | 47.29 | 0.224 |

(Example 4: Examination of mass production)

We examined the possibility of scaling up to mass production using production conditions set for a small amount of a sample. The conditions and results are summarized in the following table.

[Table 2]

|  | Automatic apparatus for small-quantity production | | Apparatus for mass production |
|---|---|---|---|
| Flow velocity | Flow velocity of flow channel for injecting nucleic acid sample:45mL/min<br>Flow velocity of flow channel for injecting lipid sample:15mL/min<br>Flow velocity of flow channel for water for secondary dilution:23.3mL/min | | |
| Injected sample volume | Nucleic acid sample:100$\mu$L<br>Lipid sample: 100$\mu$L | Nucleic acid sample:200$\mu$L<br>Lipid sample: 200$\mu$L | Nucleic acid sample:10mL<br>Lipid sample: 4mL |
| Collected volume (1 fraction) | 80$\mu$L | 240$\mu$L | 10.5mL |

(continued)

|  | Automatic apparatus for small-quantity production | | Apparatus for mass production |
|---|---|---|---|
| Composition of sample | Nucleic acid sample: nucleic acid 1.28 mg/mL (in water)<br>Lipid sample: lipid composition DOTAP:DPPC:Cholesterol:DSPE-MPEG2K=50:19.5:30:0.5, lipid concentration: 50 mg/mL (in ethanol) | | |
| Pipe | Inner diameter:0.38mm | Inner diameter:0.38mm | Inner diameter:1mm |
| Additional length of flow channel for nucleic acid relative to flow channel for lipid | 134cm | 134cm | 85cm |
| Results | Average particle diameter:62.98nm<br><br>PDI:0.115 | Average particle diameter:60.03nm diameter<br><br>PDI:0.108 | Average particle diameter:62.53nm diameter<br><br>PDI:0.106 |

It was confirmed that a larger amount could be produced using the same condition settings as when using the small amount of the sample. Performance of the resulting particles was similar under all conditions, confirming that scale-up is still possible not only for simple mixing, but also with a reaction such as particle formation.

Note that, main configurations of an automatic apparatus for small-quantity production and an apparatus for mass production were as follows.

[Table 3]

|  | Automatic apparatus for small-quantity production | Apparatus for mass production |
|---|---|---|
| Pump | LC-20AP x 3 | LTX9842 x 3 |
| Injection portion | SIL-10AP x 2 | Manual injector + sample loop |
| Collection portion | Dispenser<br><br>SHOTMASTER DS200 | Fraction collector<br><br>CHF122SC |
| Pipe for System | Inner diameter 0.381 mm, Outer diameter 1/16 inch | Inner diameter 1.0mm, Outer diameter 1/16 inch |

(Examples 5: Mixing of three components)

An additional valve was attached to the flow channel for water for secondary dilution in Example 2 to allow an acidic polymer (poly-γ-glutamic acid) to be injected into this flow channel and whether three components: a nucleic acid, a lipid, and an acidic polymer could be mixed on a single device was tested.

Flow conditions for the components are shown in the table below. For Condition 1, similar to the device configuration in Example 2, flow channels were configured so that a flow channel into which a nucleic acid sample was injected and a flow channel into which a lipid sample was injected were combined, and then a flow channel into which an acidic polymer was injected was further combined therewith. For Condition 2, flow channels were configured so that the three flow channels, i.e., the flow channel into which the nucleic acid sample was injected, the flow channel into which the lipid sample was injected, and the flow channel into which the acidic polymer was injected were combined at a single location. For each condition, a fraction with a high absorbance at OD 260, which indicates a nucleic acid concentration, was obtained, and a particle size distribution and a z-potential in that fraction were measured.

[Table 4]

| Condition | | Injected volume (mL) | Flow velocity (mL/min) |
|---|---|---|---|
| 1 | Pump A (nucleic acid) | 0.1 | 3.6 |
| | Pump B (lipid A) | 0.2 | 10.8 |
| | Pump C (acidic polymer) | 0.6 | 5.6 |
| 2 | Pump A (nucleic acid) | 0.1 | 3.6 |
| | Pump B (lipid B) | 0.2 | 10.8 |
| | Pump C (acidic polymer) | 0.6 | 5.6 |

The results are shown below. LNPs were formed by mixing the nucleic acid, the lipid, and the acidic polymer from their respective flow channels. It can be seen from measurements of the z-potential that the thus-prepared LNPs had the nucleic acid and the lipid at a center thereof and the acidic polymer in an outer shell thereof. It is understood that the method and the device of the present disclosure can test conditions for producing particles in a small amount, even if three or more components are required to be mixed with each other for forming particles.

[Table 5]

| Condition | Z-average (nm) | PDI | z-potential (mV) |
|---|---|---|---|
| 1 | 132.0 | 0.179 | -44.7 |
| 2 | 130.9 | 0.233 | -41.9 |

(Example 6: Mixing of three components, four pumps)

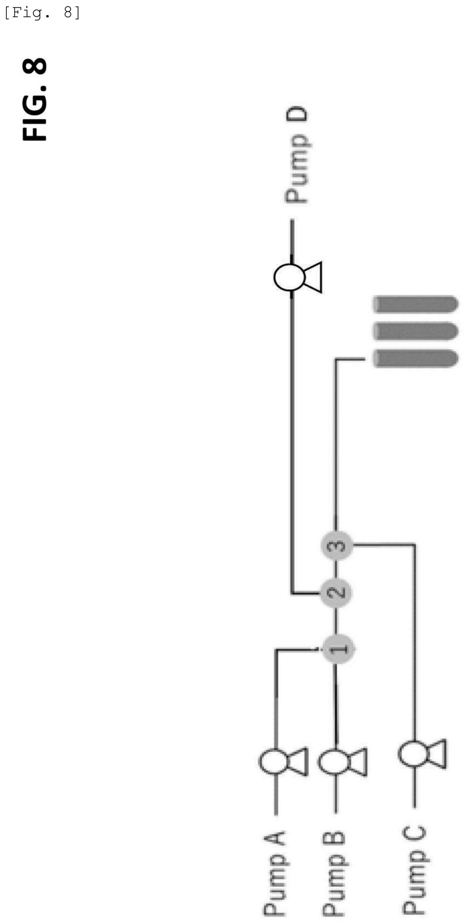

A flow channel downstream of a combination portion (1) of a flow channel through which a nucleic acid flows (pump A) and a flow channel through which a lipid flows (pump B) was combined with a flow channel through which an ethanol flows (pump D) fitted with a valve for injecting a cationic lipid (DOTAP) (combination portion 2), and further combined with a flow channel for dilution (pump C) (combination portion 3) downstream of the combination portion 2 (see FIG. 8). In this device configuration, we tested whether three components, i.e., a nucleic acid and two types of lipids could be mixed on a single device.
Flow conditions for each component are shown in the table below. For condition 2, the same amount of an ethanol solvent was injected instead of the cationic lipid. For each condition, a fraction with a high absorbance at OD 260, which indicates a nucleic acid concentration, was obtained, and a particle size distribution and a z-potential in that fraction were measured.

[Table 6]

| Condition | | Injected volume (ml) | Flow velocity (ml/min) |
|---|---|---|---|
| 1 | Pump A (nucleic acid) | 0.1 | 10.8 |
| | Pump B (lipid) | 0.2 | 3.6 |
| | Pump C (-) | 0 | 5.6 |
| | Pump D (cationic lipid) | 0.4 | 0.5 |
| 2 | Pump A (nucleic acid) | 0.1 | 10.8 |
| | Pump B (lipid) | 0.2 | 3.6 |
| | Pump C (-) | 0 | 5.6 |
| | Pump D (-) | 0.4 | 0.5 |

The results are shown below. Comparing Condition 1 with Condition 2, it is understood that addition of the cationic lipid as an additional component resulted in a positively charged surface of the LNP. It is also understood that the method and the device of the present disclosure can test conditions for producing particles in a small amount, even if a helper lipid that modifies a surface layer of the LNP is required to be sequentially mixed for forming particles.

[Table 7]

| Condition | Z-average (nm) | PDI | z-potential (mV) |
|---|---|---|---|
| 1 | 100.7 | 0.069 | 30.5 |
| 2 | 95.33 | 0.132 | -0.086 |

(Example 7: Scale-up by parallel mixing)

When lipid particles are formed, the faster a flow velocity (linear velocity), the smaller a particle diameter tends to be. In the present disclosure which assumes a high flow rate, we tested a method for reducing the linear velocity while maintaining a high flow rate so that lipid particles with various particle diameters can be formed.

The device configuration of Example 2 was modified as follows: each of the flow channel into which the nucleic acid sample was injected and the flow channel into which the lipid sample was injected were branched into two branched flow channels, the resulting two branched flow channels into which the nucleic acid sample was injected and the resulting two branched flow channels into which the lipid sample was injected were combined at two branched flow combination portions, and two flows from the two branched flow combination portions were combined at a combination portion and then further combined with the flow channel for water for secondary dilution (see FIG. 1B).

A pipe having the same inner diameter and length as in the device configuration in Example 2 was used for each branched flow channel. A test was performed at total flow rates of 30 mL/min (nucleic acid: 16.2 mL/min, lipid: 5.4 mL/min, secondary dilution: 8.4 mL/min) and 60 mL/min (nucleic acid: 32.4 mL/min, lipid: 10.8 mL/min, secondary dilution: 16.8 mL/min).

The results compared to when a flow channel configuration without branches was used are shown in the table below. The branched flow channel allowed the LNP to have an increased particle diameter while maintaining a high flow rate. Similarly, further mass production may be possible by further increasing the number of branches while maintaining conditions for forming particles.

[Table 8]

| Condition | Branched flow channel | Total flow rate (mL/min) | Z-average (nm) |
|---|---|---|---|
| 1 | No | 30.0 | 77.13 |
| 2 | No | 60.0 | 43.35 |
| 3 | Yes | 60.0 | 72.46 |

(Example 8: Installation of thermostatic bath)

A thermostatic bath (CTO-20AC, SHIMADZU CORPORATION) was attached to each of portions of the flow channel into which the nucleic acid sample was injected and the flow channel into which the lipid sample was injected before their combination portion and LNPs were formed at a heating condition of 60°C. The device configuration was the same as in Example 2.

· Nucleic acid sample: nucleic acid 1 mg/mL (in water), Injection volume 0.1 mL.
· Flow channel for injection of nucleic acid sample: flow velocity (water) 15 mL/min.
· Lipid sample: lipid for forming LNP 23 mg/mL (in ethanol), injection volume 0.2 mL. Lipid composition (DOTAP:DPPC:DSPE-Glu:Cholesterol:DSPE-MPEG2K = 20:10:10:59:1) (This lipid sample is accompanied by precipitation of the lipid under a normal storage condition at room temperature in ethanol) · Flow channel for injection of lipid sample: flow velocity (ethanol) 45 mL/min.
· Flow velocity of flow channel for water for secondary dilution: 23.3 mL/min.

A Z-average of the LNPs was 72.5 nm and a PDI was 0.075, confirming that good LNPs can be formed even when a poorly soluble lipid is used.

(Notes)

Although the present disclosure has been illustrated with reference to preferred embodiments of the present disclosure, it is understood that the scope of the present disclosure should be construed only by the claims. It is understood that the patents, patent applications, and references cited herein should be incorporated herein by reference in their entirety as if specifically set forth herein. The present application claims priority to Japanese Patent Application No. 2023-107138 filed on June 29, 2023 with the Japan Patent Office, the content of which is incorporated herein by reference in its entirety.

[Industrial Applicability]

According to the present disclosure, appropriate reaction conditions can be determined in production with consumption of only a small amount of a material.

**Claims**

1. A method for producing a formulation comprising:

    a first component; and
    a second component, the method comprising:

        preparing a series of flow channels comprising a first flow channel through which a first fluid flows at a first flow velocity, a second flow channel through which a second fluid flows at a second flow velocity, and a third flow channel through which a third fluid flows at a third flow velocity, the third flow channel being formed by combining the first flow channel and the second flow channel at a combination portion;
        injecting a sample comprising the first component into the first flow channel at a first time; and
        injecting a sample comprising the second component into the second flow channel at a second time,
        the first time and the second time being set so that the first component and the second component reach the combination portion at a time when the first component and the second component are mixed.

2. The method according to claim 1, wherein the first component is injected into a middle of the first flow channel and the second component is injected into a middle of the second flow channel.

3. The method according to claim 1 or 2, wherein a third time is set based on the first time and the second time.

4. The method according to any one of claims 1 to 3, wherein the first flow velocity or the second flow velocity exceeds about 2.4 mL/min.

5. The method according to any one of claims 1 to 4, wherein the series of flow channels comprises a fourth flow channel through which a sample comprising a third component flows; the method further comprises

    injecting the third component into the fourth flow channel at the third time; and
    the first time, the second time, and the third time are set so that the first component, the second component, and the third component reach the combination portion at a time when the first component, the second component, and the third component are mixed.

6. The method according to claim 5, wherein the third flow channel and the fourth flow channel are combined at an additional combination portion downstream of the combination portion.

7. The method according to claim 5 or 6, wherein the first flow channel, the second flow channel, and the fourth flow channel are combined at the combination portion.

8. The method according to any one of claims 1 to 7, further comprising detecting a combination state of the first component and the second component downstream of the combination portion.

9. The method according to claim 8, wherein the combination state to be detected is a state selected from the group consisting of a concentration of at least one of the first component and the second component, a particle diameter of particles in a flow, and a state of a chemical bond between the first component and the second component.

10. The method according to any one of claims 1 to 9, wherein the first flow channel is in a liquid-feeding state in the injection of the sample comprising the first component into the first flow channel and the second flow channel is in a liquid-feeding state in the injection of the sample comprising the second component into the second flow channel.

11. The method according to any one of claims 1 to 10, wherein, in the injection of the sample comprising the first component into the first flow channel and in the injection of the sample comprising the second component into the second flow channel, retention times taken for the sample comprising the first component and the sample comprising the second component to be introduced into sample injection portions and then injected into the flow channels are independently within 1 minute.

12. The method according to any one of claims 1 to 11, wherein times taken for the first component and the second component to be injected and reach the combination portion are independently within 2 seconds.

13. The method according to any one of claims 1 to 12, wherein 1000 $\mu$g or less of the first component is injected in the injection of the first component into the first flow channel and 1000 ug or less of the second component is injected in the injection of the second component into the second flow channel.

14. The method according to any one of claims 1 to 13, wherein a fourth fluid flowing through a fifth flow channel is combined with the third fluid at the additional combination portion to form a sixth flow channel through which a fifth fluid flows.

15. The method according to any one of claims 1 to 14, wherein the combination portion comprises a T-mixer, a microflow channel device, or a static mixer.

16. The method according to any one of claims 1 to 15, wherein the third flow channel comprises a mixer.

17. The method according to claim 16, wherein the mixer on the third flow channel is a static mixer.

18. The method according to any one of claims 1 to 17, wherein each of the first flow channel and the second flow channel comprises a plurality of branched flow channels, and the combination portion comprises a plurality of branched flow combination portions at which the branched flow channels from the first flow channel and the branched flow channels from the second flow channel are combined.

19. The method according to claim 18, wherein the first flow channel and/or the second flow channel is branched to form the branched flow channel.

20. The method according to claim 18 or 19, wherein the branched flow channels downstream of the branched flow combination portion combine with each other in the third flow channel.

21. The method according to any one of claims 1 to 20, further comprising removing a fraction comprising a composite of the first component and the second component from a collection portion of the third flow channel at a third time.

22. The method according to claim 21, wherein the collection portion is positioned in a middle of the third flow channel.

23. The method according to claim 21 or 22, further comprising dispensing the fraction to a receiving portion.

24. The method according to claim 23, wherein the dispensing comprises dispensing a plurality of the fractions to the receiving portion comprising a plurality of compartments.

25. The method according to claim 24, the method comprises testing the plurality of the fractions.

26. The method according to any one of claims 21 to 25, wherein a time when the fraction is removed from the collection portion is controlled based on a time and a flow velocity at which the component is injected into the flow channel.

27. An apparatus for injecting a plurality of samples into a plurality of flow channels at a time when a first component and a second component are mixed, the device comprising:

    a first flow channel through which a first fluid flows;
    a first injection portion from which a sample comprising the first component is injected into the first flow channel;
    a second flow channel through which a second fluid flows;
    a second injection portion from which a sample comprising the second component is injected into the second flow channel;

a combination portion in which the first flow channel and the second flow channel combine with each other;
a third flow channel through which a third fluid flows and which extends from the combination portion; and
a control portion,
the control portion being configured to control a time when the first injection portion injects the first component into the first flow channel and a time when the second injection portion injects the second component into the second flow channel.

28. The device according to claim 27, wherein the first injection portion is positioned in a middle of the first flow channel and the second injection portion is positioned in a middle of the second flow channel.

29. The device according to claim 27 or 28, wherein the first injection portion is a valve capable of injecting the first component into the first flow channel within 5 seconds, and the second injection portion is a valve capable of injecting the second component into the second flow channel within 5 seconds.

30. The device according to any one of claims 27 to 29, wherein at least one of the first injection portion and the second injection portion can switch flow channels within about 500 ms.

31. The device according to any one of claims 27 to 30, further comprising:

a fourth flow channel through which the third fluid flows; and
a third injection portion from which a sample comprising a third component is injected into the fourth flow channel, wherein a series of flow channels comprises the fourth flow channel, and
wherein the control portion is configured to control a time when the first injection portion injects the first component into the first flow channel, a time when the second injection portion injects the second component into the second flow channel, and a time when the third injection portion injects the third component into the fourth flow channel.

32. The device according to any one of claims 27 to 31, the device comprises a detector downstream of the combination portion.

33. The device according to any one of claims 27 to 32, wherein the first injection portion comprises a valve with a sample loop having a volume of 5 mL or less and the second injection portion comprises a valve with a sample loop having a volume of 5 mL or less.

34. The apparatus according to any one of claims 27 to 33, wherein a pump through which the first fluid and the second fluid flow is a plunger pump.

35. The apparatus according to any one of claims 27 to 34, wherein a back pressure on the pump through which the first fluid and the second fluid flow is 1 MPa or more.

36. The apparatus according to any one of claims 27 to 35, further comprising:

a fourth flow channel through which a fourth fluid flows;
an additional combination portion in which the fourth flow channel and the third flow channel combine with each other; and
a fifth flow channel through which a fifth fluid flows and which extends from the additional combination portion.

37. The apparatus according to any one of claims 27 to 36, wherein the third flow channel comprises a collection portion and, further, the collection portion comprises a collection device.

38. The apparatus according to any one of claims 27 to 37, further comprising a thermostatic bath that maintains at least a portion of the first, second, and third flow channels, the first and second injection portions, and the combination portion at a constant temperature.

39. The apparatus according to claim 37 or 38, wherein the control portion further controls a collection time of the collection device.

40. The apparatus according to claim 39, wherein the control portion controls at least one of a collection start time and a time interval between collections of the collection device.

41. The method according to claims 38 to 40, wherein the collection portion is positioned in a middle of the third flow channel.

42. The apparatus according to any one of claims 38 to 41, wherein the collection device comprises a valve.

43. The apparatus according to claim 42, wherein the valve is a slide solenoid valve.

44. The apparatus according to any one of claims 38 to 43, further comprising a receiving portion that receives a fraction removed from the collection device.

45. The apparatus according to claim 44, wherein the receiving portion comprises a plurality of compartments that receive a plurality of fractions.

46. A program for causing an apparatus comprising a processor to implement a method for injecting a plurality of components into a plurality of flow channels connected to a combination portion, the method comprising:

   receiving information about a first flow channel and information about a second flow channel;
   determining a first time at which a sample comprising a first component is injected into the first flow channel and a second time at which a sample comprising a second component is injected into the second flow channel based on the information about the first flow channel and the information about the second flow channel, the first time and the second time being times set so that the first component injected into the first flow channel and the second component injected into the second flow channel reach a combination portion at a time when the first component and the second component are mixed;
   instructing an injection device to inject the sample comprising the first component into the first flow channel at the first time; and
   instructing an injection device to inject the sample comprising the second component into the second flow channel at the second time.

47. The program according to claim 46, wherein the method further comprises

   receiving information about a third flow channel downstream of the combination portion;
   determining a third time at which a fraction is removed from the third flow channel based on the information about the third flow channel; and
   instructing a collection device to remove the fraction from the third flow channel at the third time.

48. A recording medium storing a program for causing an apparatus comprising a processor to implement a method for injecting a plurality of components into a plurality of flow channels connected to a combination portion, the method comprising:

   receiving information about a first flow channel and information about a second flow channel;
   determining a first time at which a sample comprising a first component is injected into the first flow channel and a second time at which a sample comprising a second component is injected into the second flow channel based on the information about the first flow channel and the information about the second flow channel, the first time and the second time being times set so that the first component injected into the first flow channel and the second component injected into the second flow channel reach a combination portion at a time when the first component and the second component are mixed;
   instructing an injection device to inject the sample comprising the first component into the first flow channel at the first time; and
   instructing an injection device to inject the sample comprising the second component into the second flow channel at the second time.

49. The recording medium according to claim 48, wherein the method further comprises

   receiving information about a third flow channel downstream of the combination portion;
   determining a third time at which a fraction is removed from the third flow channel based on the information about the third flow channel; and
   instructing a collection device to remove the fraction from the third flow channel at the third time.

50. A method for screening a condition for producing drug-loaded lipid particles, the method comprising:

preparing a series of flow channels comprising a first flow channel through which a first fluid flows at a first flow velocity, a second flow channel through which a second fluid flows at a second flow velocity, and a third flow channel through which a third fluid flows at a third flow velocity, the third flow channel being formed by combining the first flow channel and the second flow channel at a combination portion;

(1) injecting a sample comprising a candidate lipid into the first flow channel at a first time;
(2) injecting a sample comprising a candidate drug into the second flow channel at a second time;
(3) removing a fraction comprising particles of the candidate lipid from a collection portion of the third flow channel at a third time; and

evaluating the particles of the candidate lipid,
the first time and second time being set so that the candidate lipid and the candidate drug simultaneously reach the combination portion.

51. The method according to claim 50, further comprising preparing a plurality of the particles of the candidate lipid, comparing the plurality of the particles of the candidate lipid, and selecting hit lipid particles by repeating (1) to (3).

52. A method for producing the hit lipid particles according to claim 51 under the condition set in the method for screening according to claim 50.

53. The method for producing the hit lipid particles according to claim 52, wherein a frequency with which (1) to (3) are performed is increased, thereby increasing an amount of the hit lipid particles to be produced.

FIG. 1A

EP 4 736 997 A1

[Fig. 1B]

**FIG. 1B**

[Fig. 2]

**FIG. 2**

111

110

[Fig. 3A]

**FIG. 3A**

30

Control unit

31

Control portion

32

Detection portion

Thermostatic bath

Thermostatic bath

**3a** Pump

**3b** Pump

**3c** Pump

Input portion

Display portion

41

42

Memory portion

43

Interface

44

Pressure sensor

Pressure sensor

Temperature sensor

Temperature sensor

[Fig. 3B]

FIG. 3B

[Fig. 4]

FIG. 4

[Fig. 5]

# FIG. 5

FIG. 6

EP 4 736 997 A1

[Fig. 7]

FIG. 7

[Fig. 8]

EP 4 736 997 A1

**FIG. 8**

## INTERNATIONAL SEARCH REPORT

| International application No. |
|---|
| **PCT/JP2024/023467** |

### A. CLASSIFICATION OF SUBJECT MATTER

*B01F 23/45*(2022.01)i; *A61K 9/127*(2006.01)i; *B01F 25/43*(2022.01)i; *B01F 33/301*(2022.01)i; *B01F 35/22*(2022.01)i; *B01F 35/71*(2022.01)i; *B01F 35/75*(2022.01)i; *B01F 35/92*(2022.01)i; *B01F 35/213*(2022.01)i; *B01F 35/221*(2022.01)i; *B01J 2/10*(2006.01)i; *C12N 15/88*(2006.01)i; *B01F 101/22*(2022.01)n

FI:  B01F23/45; B01J2/10 A; B01F25/43; B01F33/301; B01F35/221; B01F35/213; B01F35/92; B01F35/75; B01F35/71; B01F35/22; A61K9/127; C12N15/88 Z; B01F101:22

According to International Patent Classification (IPC) or to both national classification and IPC

### B. FIELDS SEARCHED

Minimum documentation searched (classification system followed by classification symbols)

B01F21/00-25/90; B01J2/00-2/30; B01F29/00-33/87; A61K9/00-9/72; A61K47/00-47/69; C12N15/00-15/90

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2013-512438 A (OPKO DIAGNOSTICS LLC) 11 April 2013 (2013-04-11) entire text, all drawings | 1-53 |
| A | JP 2019-042713 A (HITACHI, LTD.) 22 March 2019 (2019-03-22) entire text, all drawings | 1-53 |
| A | JP 2006-247534 A (BUSSAN NANOTECH RESEARCH INSTITUTE INC.) 21 September 2006 (2006-09-21) entire text, all drawings | 1-53 |
| A | JP 2003-190751 A (MINOLTA CO., LTD.) 08 July 2003 (2003-07-08) entire text, all drawings | 1-53 |
| A | JP 2007-043998 A (TOSOH CORPORATION) 22 February 2007 (2007-02-22) entire text, all drawings | 1-53 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **26 August 2024** | **10 September 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**
Information on patent family members

International application No.

**PCT/JP2024/023467**

| Patent document cited in search report | | | Publication date (day/month/year) | Patent family member(s) | | | Publication date (day/month/year) |
|---|---|---|---|---|---|---|---|
| JP | 2013-512438 | A | 11 April 2013 | US | 2011/0120562 | A1 | |
| | | | | whole document | | | |
| JP | 2019-042713 | A | 22 March 2019 | US | 2020/0282376 | A1 | |
| | | | | whole document | | | |
| JP | 2006-247534 | A | 21 September 2006 | (Family: none) | | | |
| JP | 2003-190751 | A | 08 July 2003 | (Family: none) | | | |
| JP | 2007-043998 | A | 22 February 2007 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016024510 A

- JP 2023107138 A

**Non-patent literature cited in the description**

- **GREGORY GREGORIADIS**. Liposome Technology: Liposome Preparation and Related Techniques. CRC Press, 12 September 2006